# EUROPEAN PATENT SPECIFICATION

(11) **EP 4 151 268 B1**
(45) Date of publication and mention of the grant of the patent: **25.06.2025**
(21) Application number: 21804124.2
(22) Date of filing: 11.05.2021
(51) Int. Cl.: A61M 35/00, A61F 9/00

(54) **DEVICE FOR CAUSING TEARS TO BE SECRETED**
VORRICHTUNG ZUR VERANLASSUNG DER SEKRETIERUNG VON TRÄNEN
DISPOSITIF POUR PROVOQUER LA SÉCRÉTION DE LARMES

(30) Priority: 11.05.2020 JP 2020083525
(43) Date of publication of application: 22.03.2023
(73) Proprietor: Provigate Inc., Tokyo 113-0033 (JP)
(72) Inventor: NISHI Mitsumi, Tokyo 113-0033 (JP); MIYAUCHI Noriko, Tokyo 113-0033 (JP)
(74) Representative: Michalski Hüttermann & Partner Patentanwälte mbB
(86) International application number: PCT/JP2021/017957
(87) International publication number: WO 2021/230260

(56) References cited:
- WO-A1-2005/067907
- JP-A- 2019 507 652
- US-A- 5 578 021
- US-A1- 2007 282 012
- US-B1- 6 251 952

## Description

### Technical Field

The present disclosure relates to a device for stimulating tear secretion.

### Background Art

Tear fluid may be collected by, for example, pressing test paper against the surface of an eyeball so that the test paper absorbs the tear fluid.

It is desirable to perform tear fluid collection efficiently. However, the method of pressing the test paper against the surface of the eyeball may involve a painful stimulus on the eyeball. In addition, tear fluid secretion is not readily controllable by a subject from which tear fluid is to be collected, and it may take a long time to collect a sufficient amount of tear fluid. Therefore, the tear fluid collection may be stressful for the subject from which tear fluid is collected. It is desirable to perform tear fluid collection efficiently with less stress on the subject.

US2007/282012 discloses a device for stimulating tear secretion according to the preamble of claim 1.

### Summary of Invention

The invention is defined by the independent claim 1. Here, it is recognized, for example, without limitation, that efficient tear fluid collection with less stress on the subject is desired.

Some embodiments of the present disclosure provide a device for stimulating tear secretion. In some embodiments, the device includes a container body. In some embodiments, the container body includes a rim capable of coming into close contact with a face in a region around an eye. In some embodiments, the container body defines a gas-receiving space. In some embodiments, the gas-receiving space is capable of being filled with gas generated by an eye irritant.

The above-described embodiments, for example, enable efficient tear fluid collection with less stress on the subject.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### Brief Description of Drawings

[Fig. 1] Fig. 1 is a schematic illustration of a device according to an embodiment.
[Fig. 2] Fig. 2 illustrates the device according to the embodiment in Fig. 1 viewed from an opening.
[Fig. 3] Fig. 3 is a schematic illustration of a device according to an embodiment.
[Fig. 4] Fig. 4 illustrates the device according to the embodiment in Fig. 3 viewed from an opening.
[Fig. 5] Fig. 5 is a schematic illustration of a device according to an embodiment.
[Fig. 6] Fig. 6 illustrates the device according to the embodiment in Fig. 5 viewed from an opening.
[Fig. 7] Fig. 7 is a schematic illustration of a device according to an embodiment.
[Fig. 8] Fig. 8 illustrates the device according to the embodiment in Fig. 7 viewed from an opening.
[Fig. 9] Fig. 9 is a schematic illustration of a device according to an embodiment.
[Fig. 10] Fig. 10 illustrates the device according to the embodiment in Fig. 9 viewed from an opening.
[Fig. 11] Fig. 11 is a schematic illustration of a device according to an embodiment.
[Fig. 12] Fig. 12 is a schematic illustration of a device according to an embodiment.
[Fig. 13] Fig. 13 is a schematic illustration of a device according to an embodiment.
[Fig. 14] Fig. 14 is a schematic illustration of a device according to an embodiment.
[Fig. 15] Fig. 15 is a schematic illustration of a device according to an embodiment.
[Fig. 16] Fig. 16 is a schematic illustration of a device according to an embodiment.
[Fig. 17] Fig. 17 is a schematic illustration of a device according to an embodiment.
[Fig. 18] Fig. 18 is a schematic illustration of a device according to an embodiment.
[Fig. 19] Fig. 19 is a schematic illustration of a device according to an embodiment.
[Fig. 20] Fig. 20 is a schematic illustration of a device according to an embodiment.
[Fig. 21] Fig. 21 is a schematic illustration of a device according to an embodiment.
[Fig. 22] Fig. 22 is a schematic illustration of a device according to an embodiment.

### Description of Embodiments

In some embodiments, a device stimulates tear fluid secretion. The device may or may not collect the tear fluid produced by secretion.

In some embodiments, when the device does not collect the tear fluid produced by secretion stimulated by the device, the produced tear fluid may be collected by other collecting means. The other collecting means may be, for example, a container that collects the tear fluid falling from the eye or a member other than the container. The other collecting means may be, for example, a dropper used to collect the tear fluid produced by secretion in the eye by suction or a member other than the dropper.

In some embodiments, the tear fluid is produced by secretion in the eye of a subject. In some embodiments, the subject may include, or may be, a human.

In some embodiments, the subject may include, or may be, a non-human animal. The non-human animal may include, or may be, a mammal. Examples of non-human animals include, but are not limited to, working animals, livestock animals, pet animals, and wild animals.

In some embodiments, the device includes a container body. In some embodiments, the container body includes a rim capable of coming into close contact with a face in a region around an eye. In some embodiments, when tear fluid secretion is to be stimulated, the rim of the container body is brought into close contact with the face of the subject in the region around the eye.

The term "region around the eye" used herein generally refers to a portion of the face positioned in a region around the eye. In some aspects, the region around the eye may, for example, include one or more of a region closer to the nose than the inner corner of the eye, a region outside the outer corner of the eye and close to the ear, the upper eyelid, a region above the upper eyelid, the lower eyelid, and a region below the lower eyelid. In some aspects, when the subject's head is in a position for tear fluid secretion in the eye, a portion of the face with which the rim is capable of coming into close contact may include, or may be, a portion of the subject's face below the eye.

In some embodiments, the container body defines a gas-receiving space. In some embodiments, the device may have a gas-receiving space that is provided in the container body and to which gas generated by an eye irritant is supplied.

The term "gas-receiving space" used herein generally refers to a space capable of being filled with gas generated by an eye irritant.

In some embodiments, the eye irritant may generate gas that irritates the eye to stimulate tear fluid secretion in the eye. In some embodiments, the eye irritant may include, or may be, a substance that generates a component thereof by reaction.

In some embodiments, an active ingredient of the eye irritant may include, or may be, a lachrymatory component or a lachrymatory factor. In some embodiments, the eye irritant may include, or may be, some (one of more types) of a plurality of substances necessary to generate a lachrymatory substance.

In some embodiments, the active ingredient of the eye irritant may include, or may be, a naturally derived substance. In some embodiments, the active ingredient of the eye irritant may include, or may be, a substance extracted from a plant of the genus Allium. Examples of plants of genus Allium include, but are not limited to, bulb onion (Allium cepa), Welsh onion (Allium fistulosum), garlic (Allium sativum), and Sicilian garlic (Allium siculum), and one or more thereof may be included.

In some embodiments, the active ingredient of the eye irritant may include, or may be, a synthetically produced substance. In some embodiments, the eye irritant may include, or may be, a chemical substance. In some embodiments, the eye irritant may include, or may be, lachrymatory gas or a lachrymatory agent. In some embodiments, the eye irritant may be volatile. In some embodiments, the eye irritant may include, or may be, an eye irritating component. In some embodiments, the eye irritant may be benzyl bromide (α-bromotoluene, bromomethylbenzene). In some embodiments, the eye irritant may be benzyl chloride (α-chlorotoluene, chloromethylbenzene), and one or more thereof may be included.

In some embodiments, the active ingredient of the eye irritant may include, or may be, a compound having a structure called thioalkanal S-oxide at the end of an alkyl chain, or a compound analogous thereto. In some embodiments, the active ingredient of the eye irritant may be a compound represented by R-CH=S-O. Examples of the compound include, but are not limited to, thioethanal S-oxide (R = C1), thiopropanal S-oxide (R = C2), thiobutanal S-oxide (R = C3), and thiohexanal S-oxide (R = C4).

In some embodiments, the eye irritant may include, or may be, syn-propanethial-S-oxide (may be referred to also as C₃H₆OS, thiopropanal S-oxide, or 1-sulfinylpropane). In some embodiments, the eye irritant may include, or may be, a substance from which syn-propanethial-S-oxide is synthesized. In some embodiments, the eye irritant may be syn-propanethial-S-oxide generated by reaction of substances upon use.

In some embodiments, for example, alliinase (supported by cellulose in a dry state), which is a type of enzyme, may be used to break down amino acid sulfoxide (substrate) to generate sulfenic acid. In some embodiments, lachrymatory factor synthase (LFS) may be used to convert the generated 1-propenesulfenic acid into syn-propanethial-S-oxide. In some embodiments, alliinase and LFS may be placed in a container. In some embodiments, these enzymes may be supported by a carrier in a dry state in the container.

In some embodiments, for example, syn-propanethial-S-oxide may be generated by using one or more of PRENCSO (trans-1-propenyl-L-cysteine sulfoxide), alliinase, and LFS. In some embodiments, for example, one or more of PRENCSO, alliinase, and LFS may be supported in the container and caused to react with the rest upon use.

In some embodiments, for example, the active ingredient of the eye irritant may include, or may be, syn-butanethial-S-oxide, C₄H₈OS.

In some embodiments, for example, the eye irritant may be various other types of lachrymatory gas. The lachrymatory gas may be used within a range in which safety is ensured. For example, the lachrymatory gas may be diluted. For example, the lachrymatory gas may be modified to enhance safety. Examples of the lachrymatory gas include, but are not limited to: emetics, such as adamsite; sternutatories, such as diphenylcyanoarsine (DC) and diphenylchloroarsine (DA); and lachrymatory agents, such as bromobenzylcyanide, chloroacetophenone, chlorobenzylidene malononitrile, and dibenzoxazepine. One or more of these examples may be included. The lachrymatory gas may be pepper spray, and capsaicin (referred to also as oleoresin capsicum or an OC agent), which constitutes or is the main component of the pepper spray, may be included.

In some embodiments, the device may further include a fiber structure that supports the eye irritant and that is disposed in an irritant placement section. Examples of the fiber structure that supports the eye irritant include, but are not limited to, cellulose, gauze, non-woven fabric, and sponge. The fiber structure that supports the eye irritant may be in various shapes. Examples of shapes of the fiber structure include, but are not limited to, a circular shape, a sheet shape, a string shape, a round shape, and a spherical shape. The fiber structure may be, for example, cotton-like or fluffy. Accordingly, for example, the surface area is substantially increased, so that the efficiency of vaporization of the lachrymatory component and the efficiency of permeation of the substrate solution are increased. In some embodiments, the eye irritant may be liquid and sealed in a bag. In some embodiments, the device may include no fiber structure that supports the eye irritant, and components of the eye irritant may be supported in the container body in a dry state. In some embodiments, the eye irritant may be prepared in a solid state, for example, in the form of a tablet or powder. For example, the eye irritant may be prepared in the form of a mixture with an excipient.

In some embodiments, the eye irritant may include at least a portion of an enzyme and a substrate that react to generate gas, or may be an enzyme and a substrate that react to generate gas. In some embodiments, the enzyme may be disposed in the irritant placement section in a dry state, and the substrate may be added to the enzyme in the form of a solution. In some embodiments, the enzyme may be alliinase and lachrymatory factor synthase (LFS). In some embodiments, the substrate may be PRENCSO. In some embodiments, a solution of PRENCSO, which is the substrate, may be dropped onto the above-mentioned enzymes. These substances may react to generate the active ingredient of the eye irritant.

In some embodiments, the container body may or may not have a cup shape with an opening. In some embodiments, when the container body does not have a cup shape, the container body may, for example, have any shape capable of receiving the tear fluid. The container body may have, for example, a plate shape, a dish shape with a concave surface inside the rim, or a tubular shape with a bottom portion. In some embodiments, the container body may include a bottom portion disposed at other side in a first direction and a peripheral wall extending from the peripheral edge of the bottom portion toward one side in the first direction.

In some embodiments, the opening of the container body may open toward the one side in the first direction or in a direction other than toward the one side in the first direction. The term "first direction" used herein generally refers to a direction in which the opening of the container body opens.

In some embodiments, when the container body has the opening, the rim may extend continuously along the entire periphery of the opening or along only a portion of the opening. In some embodiments, the container body may or may not define a substantially closed space between the container body and the eye when the rim of the container body is in close contact with the face in the region around the eye. In some embodiments, when the container body does not define a substantially closed space between the container body and the eye, a partially open space may be formed between the container body and the eye.

In some embodiments, the device may or may not further include an irritant placement section that communicates with the gas-receiving space and in which the eye irritant is placed. In some embodiments, the irritant placement section may or may not be disposed in at least a portion of the gas-receiving space. In some embodiments, the irritant placement section may be separated from the gas-receiving space. In some embodiments, the irritant may be disposed at a position closest to the iris or pupil of the user when the device is in use. According to one theory, the lachrymatory effect is greatest on these portions of the eyeball. For example, the irritant placement section may be situated substantially at the center of the bottom portion of the container, or at or near the center of volume of the container.

In some embodiments, a tear-fluid-receiving section in which the tear fluid produced by secretion in the eye is collected may or may not be provided in the container body.

In some embodiments, the container body may or may not be provided with a partition wall that partitions the interior of the container body into a gas-receiving section and the tear-fluid-receiving section. In some embodiments, when the partition wall is not provided, the gas-receiving section and the tear-fluid-receiving section may be provided in the container body without being partitioned from each other.

In some embodiments, when the gas-receiving section and the tear-fluid-receiving section are provided in the container body without being partitioned from each other, the irritant placement section may be disposed on the bottom portion of the container body or on a portion other than the bottom portion of the container body. In some embodiments, the irritant placement section may be disposed above the bottom portion. In some embodiments, assuming that the irritant placement section is disposed on the bottom portion of the container body, the bottom portion may or may not be inclined toward the other side in the first direction from a bottom intermediate section toward a first end and a second end of the container body, the bottom intermediate section being positioned between the first end and the second end. The first end and the second end are respectively positioned adjacent to the outer corner and the inner corner of the eye when the rim is in close contact with the face in the region around the eye.

In some embodiments, the irritant placement section may or may not be disposed on the bottom portion at a location closer to the second end than the bottom intermediate section. In some embodiments, when the bottom portion includes a base that bulges toward the one side in the first direction from a peripheral edge of the bottom portion toward an inner region of the bottom portion, the irritant placement section may or may not be disposed on the base. In some embodiments, the bottom portion may be removably attachable to the peripheral wall, and the irritant placement section may be disposed on the bottom portion or on a portion other than the bottom portion.

In some embodiments, the irritant placement section may or may not be disposed on an inner peripheral surface of the peripheral wall. In some embodiments, the irritant placement section may include a holder body that holds the eye irritant and a connection member having one end connected to the holder body and the other end connected to the container body. In some embodiments, the container body may include a removably attachable member that is removably attachable to the opening of the container body, and the irritant placement section may be disposed on a surface of the removably attachable member that faces the inner region of the container body when the removably attachable member is attached to the opening.

The bottom portion may be removably attached to the peripheral wall in various forms. For example, the bottom portion and the peripheral wall may each have a magnet and thereby be removably attachable to each other. Examples of forms of removable attachment between the bottom portion and the peripheral wall include, but are not limited to, magnets, both-sided tape, a hook-and-loop fastener, fitting, engagement, and insertion (for example, a portion of the bottom portion may be slid into a slit formed in the peripheral wall). Other forms may also be employed.

In some embodiments, the partition wall may be disposed between the first end and the second end that are respectively positioned adjacent to the outer corner and the inner corner of the eye when the rim is in close contact with the face in the region around the eye. The partition wall may or may not partition the interior of the container body into a first section adjacent to the first end and a second section adjacent to the second end. In some embodiments, when the partition wall partitions the interior of the container body into the first section and the second section, the first section may constitute the tear-fluid-receiving section, and the second section may constitute the gas-receiving space. In some embodiments, the first section may constitute the gas-receiving space, and the second section may constitute the tear-fluid-receiving section.

In some embodiments, the partition wall may be disposed between an upper end and a lower end of the container body, the upper end and the lower end being respectively positioned adjacent to an upper eyelid and a lower eyelid when the rim is in close contact with the face in the region around the eye. The partition wall may or may not partition the interior of the container body into an upper section adjacent to the upper end and a lower section adjacent to the lower end. In some embodiments, when the partition wall partitions the interior of the container body into the upper section and the lower section, the upper section may constitute the tear-fluid-receiving section, and the lower section may constitute the gas-receiving space. In some embodiments, the upper section may constitute the gas-receiving space, and the lower section may constitute the tear-fluid-receiving section.

In some embodiments, the entirety of an outer peripheral edge of the partition wall may be joined to an inner peripheral surface of the container body so that a sealed space is formed between the partition wall and the inner peripheral surface of the container body. The sealed space may constitute the irritant placement section, and the partition wall may have a ventilation hole through which regions on both sides of the partition wall communicate with each other.

In some embodiments, the partition wall may have a tubular shape that extends from the bottom portion toward the one side in the first direction, and the irritant placement section may be disposed inside the partition wall having the tubular shape.

In some embodiments, the partition wall may be disposed on the peripheral wall at an intermediate position in the first direction and extends along a plane that crosses the first direction to partially partition the inner space of the container body. In some embodiments, when the partition wall partially partitions the inner space of the container body, the irritant placement section may be disposed closer to the bottom portion than the partition wall in the first direction, and the tear-fluid-receiving section may be disposed further away from the bottom portion than the partition wall in the first direction. In some embodiments, when the partition wall partially partitions the inner space of the container body, the irritant placement section may be disposed further away from the bottom portion than the partition wall in the first direction, and the tear-fluid-receiving section may be disposed closer to the bottom portion than the partition wall in the first direction. In some embodiments, the partition wall may have a communicating portion through which regions closer to and further away from the bottom portion than the partition wall in the first direction communicate with each other in the inner space of the container body, and the communicating portion may be disposed adjacent to the inner corner of the eye when the rim of the container body is in close contact with the face in the region around the eye. In some embodiments, the communicating portion may be disposed adjacent to the outer corner of the eye when the rim of the container body is in close contact with the face in the region around the eye. In some embodiments, the partition wall may or may not be inclined toward the bottom portion in the first direction from the communicating portion toward the peripheral wall. In some embodiments, the partition wall may be inclined away from the bottom portion in the first direction from the communicating portion toward the peripheral wall.

In some embodiments, the device may or may not include a tear fluid container that is integrated with the container body and in which the tear fluid received by the tear-fluid-receiving section is collected. In some embodiments, the tear fluid container may or may not have a tubular shape with a bottom that extends outward from the opening of the container body. In some embodiments, when the tear fluid container does not have a tubular shape with a bottom, the shape of the tear fluid container is, for example, but not limited to, a hollow spherical shape or a hollow box shape with an opening that communicates with the container body. In some embodiments, the tear fluid container may be formed non-integrally with the container body. In some embodiments, the tear fluid container may be provided separately from the container body. In some embodiments, when the tear fluid container is provided separately from the container body, a connecting tube that connects the container body to the tear fluid solution may be additionally provided. In some embodiments, the tear fluid container may be provided separately from the container body and configured to allow the tear solution to be transferred to the fluid container from the container body.

In some embodiments, the tear fluid container may be integrated with the container body and disposed on the container body at a location adjacent to the outer corner of the eye when the rim of the container body is in close contact with the face in the region around the eye, and the tear fluid container may have a container opening that opens toward the inner corner of the eye. In some embodiments, the tear fluid container may be integrated with the container body and disposed on the container body at a location other than the location adjacent to the outer corner of the eye when the rim of the container body is in close contact with the face in the region around the eye. In some embodiments, the tear fluid container may have a container opening that opens in a direction other than toward the inner corner of the eye. In some embodiments, the tear fluid container may have a container opening that opens toward the inner region of the container body.

In some embodiments, the container body may have an access opening through which the inside of the container body is accessible from the outside when the rim of the container body is in close contact with the face in the region around the eye. Examples of the use of the access opening include, but are not limited to, collection of the tear fluid and introduction of the eye irritant.

In some embodiments, at least a portion or the entirety of the container body may be made of a material having transparency characteristics that allow optical observation of the inside of the container body from the outside. Examples of the material having transparency characteristics that allow optical observation of the inside of the container body from the outside include, but are not limited to, resin materials and glass materials. In some embodiments, the material having transparency characteristics that allow optical observation of the inside of the container body from the outside may be transparent so that the amount of tear fluid accumulated in the container body is observable visually or with a camera from the outside of the container body. In some embodiments, a camera may be placed in the container body. In some embodiments, necessary light may be introduced into the container from the outside. In some embodiments, necessary light may be emitted in the container body.

In some embodiments, the device may include two of the container bodies. In some embodiments, the device may include a connector that connects inner-corner end portions of the two container bodies to each other, the inner-corner end portions being adjacent to the inner corners of the eyes when the rims of the two container bodies are in close contact with the face in the regions around the eyes. In some embodiments, the device may further include a retainer capable of retaining the or each container body on the head of the subject such that the rim of the container body is in close contact with the face in the region around the eye. In some embodiments, a retainer may be provided on outer-corner end portions of the two container bodies, the outer-corner end portions being adjacent to the outer corners of the eyes when the rims of the two container bodies are in close contact with the face in the regions around the eyes. The retainer is capable of retaining the two container bodies on the head of the subject. The shape of the retainer may be, for example, but not limited to, the shape of a temple of eyeglasses such that the retainer may be placed on each ear of the subject, or a band shape such that the retainer may be wrapped around the back of the subject's head.

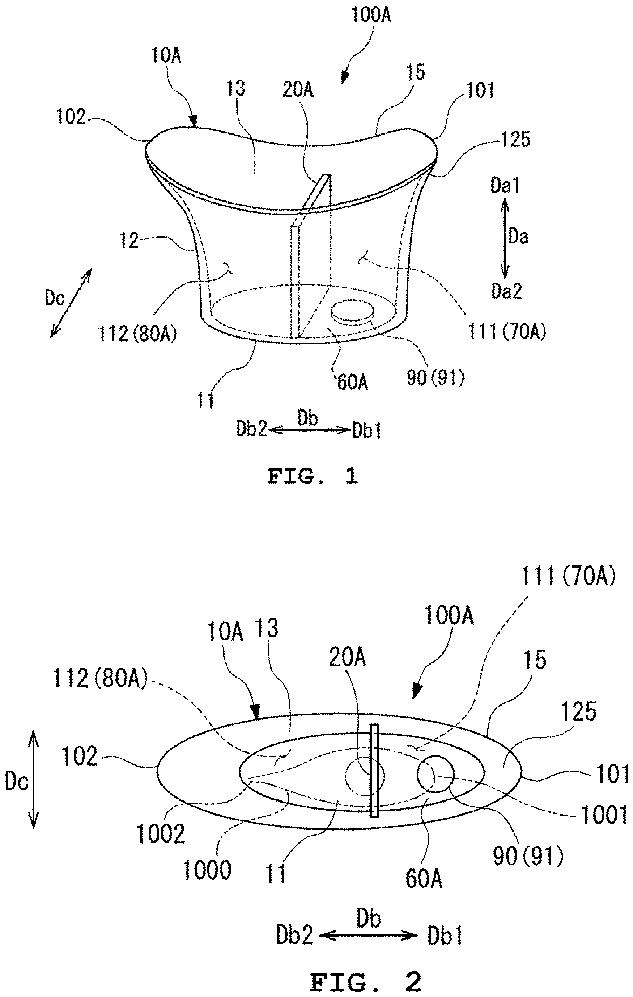

Figs. 1 and 2 illustrate a device 100A according to an embodiment. The device 100A illustrated in Figs. 1 and 2 includes a container body 10A and a partition wall 20A.

The container body 10A includes a bottom portion 11 and a peripheral wall 12. The container body 10A has an opening 13 that opens toward one side Da1 in a first direction Da. The container body 10A is made of a material having transparency characteristics that allow optical observation of the inside of the container body 10A from the outside. For example, the container body 10A is made of a transparent or translucent resin material.

The bottom portion 11 is disposed at an end of the container body 10A at other side Da2 in the first direction Da. The first direction Da is a direction in which the opening 13 opens. The first direction Da is a direction connecting the opening 13 and the bottom portion 11. The bottom portion 11 has, for example, a plate shape and extends along a plane that crosses (is substantially orthogonal to) the first direction Da. The bottom portion 11 has an elliptical shape when viewed in the first direction Da. The major axis of the bottom portion 11 having the elliptical shape extends in a second direction Db orthogonal to the first direction Da. The minor axis of the bottom portion 11 extends in a third direction Dc orthogonal to the first direction Da and the second direction Db. The bottom portion 11 may also have, for example, an oval shape, a circular shape, or a polygonal shape when viewed in the first direction Da.

The peripheral wall 12 is formed to extend from the peripheral edge of the bottom portion 11 toward the one side Da1 in the first direction Da. The peripheral wall 12 has a tubular shape that extends in the first direction Da. The peripheral wall 12 is formed such that the inner diameter thereof gradually increases from the bottom portion 11 at the other side Da2 in the first direction Da toward the one side Da1. A peripheral edge 125 of the peripheral wall 12 at an end at the one side Da1 in the first direction Da broadens in a flared shape (bell shape) toward the one side Da1 in the first direction Da. A rim 15 is formed along the peripheral edge 125 of the peripheral wall 12. The rim 15 is capable of coming into close contact with the face of a subject in a region around the subject's eye when the device 100A is used. A substantially closed space is formed between the container body 10A and an eye 1000 when the rim 15 is in close contact with the face in the region around the eye 1000. It is not necessary that the rim 15 come into complete contact with the face in the region around the eye 1000 to form a completely closed space. A small gap may be present between the rim 15 and the face in the region around the eye 1000 as long as tear fluid can be collected in the container body 10A.

As illustrated in Fig. 2, the container body 10A is used with the rim 15 in close contact with the face of the subject in the region around the eye 1000. The container body 10A is positioned such that the second direction Db substantially aligns with a direction connecting an inner corner 1001 and an outer corner 1002 of the subject's eye 1000. The container body 10A has a first end 101 and a second end 102. The first end 101 is positioned adjacent to the inner corner 1001 when the rim 15 is in close contact with the face in the region around the eye 1000. The second end 102 is positioned adjacent to the outer corner 1002 when the rim 15 is in close contact with the face in the region around the eye 1000.

The partition wall 20A partitions the interior of the container body 10A into a plurality of spaces. The partition wall 20A is disposed between the first end 101 and the second end 102. The partition wall 20A is disposed at an intermediate location between the first end 101 and the second end 102 in the second direction Db. The partition wall 20A may also be disposed closer to the first end 101 or the second end 102 than the intermediate location between the first end 101 and the second end 102 in the second direction Db. The partition wall 20A extends along a plane substantially orthogonal to the second direction Db. The partition wall 20A has an outer edge joined to the bottom portion 11 and the peripheral wall 12 of the container body 10A. The partition wall 20A partitions the interior of the container body 10A into a first section 111 adjacent to the first end 101 and a second section 112 adjacent to the second end 102.

A gas-receiving space 70A and a tear-fluid-receiving section 80A are provided in the container body 10A. The partition wall 20A partitions the interior of the container body 10A into the gas-receiving space 70A and the tear-fluid-receiving section 80A. In the present disclosure, the first section 111 constitutes the gas-receiving space 70A. The second section 112 constitutes the tear-fluid-receiving section 80A.

The gas-receiving space 70A is defined by the container body 10A. An irritant placement section 60A is disposed in the container body 10A. An eye irritant 90 is disposed in the irritant placement section 60A. In the present disclosure, the eye irritant 90 is disposed in the gas-receiving space 70A. Accordingly, the irritant placement section 60A is disposed in at least a portion of the gas-receiving space 70A. Thus, the irritant placement section 60A communicates with the gas-receiving space 70A.

The eye irritant 90 generates gas that irritates the eye to stimulate tear fluid secretion in the eye. The eye irritant 90 generates a component that stimulates tear fluid secretion in the eye by reaction.

In the embodiment of the present disclosure, an active ingredient of the eye irritant 90 contains a substance extracted from a plant of the genus Allium, which is a naturally derived substance. In the embodiment of the present disclosure, the eye irritant 90 includes, for example, alliinase and LFS, which are types of enzymes. The eye irritant 90 is, for example, supported in a dry state by cellulose, which serves as a fiber structure 91.

In the embodiment of the present disclosure, the eye irritant 90 includes PRENCSO that serves as a substrate and that is supported in a dry state by the fiber structure 91.

The enzymes in the eye irritant 90 react with a solution (aqueous solution) of the substrate to generate syn-propanethial-S-oxide. Gas containing the generated syn-propanethial-S-oxide irritates the eye to stimulate tear fluid secretion.

In some embodiments, a mixture of a substrate and an enzyme may be provided, and water may be added thereto. In some embodiments, the eye irritant 90 may include an enzyme and a substrate.

To collect tear fluid from the subject's eye by using the device 100A, the eye irritant 90 is placed in the irritant placement section 60A of the container body 10A. The eye irritant 90 may also be, for example, fixed to an inner peripheral surface of the container body 10A in advance with an adhesive or the like. When a substrate solution or water is dropped onto the eye irritant 90 in the container body 10A, the gas-receiving space 70A is filled with gas generated by the eye irritant 90. The container body 10A is positioned such that the opening 13 thereof faces toward the subject's eye 1000, and the rim 15 is brought into close contact with the face in the region around the eye 1000. The subject tilts their head so that the outer corner 1002 of the eye 1000 against which the container body 10A is pressed is lowered with respect to the inner corner 1001.

The gas in the gas-receiving space 70A comes into contact with the subject's eyeball and irritates the eye 1000. As a result, tear fluid is produced by secretion in the eye 1000. The produced tear fluid flows out of the eye 1000 from the outer corner 1002 at a lower position. The tear fluid that has flowed out of the eye 1000 flows into the tear-fluid-receiving section 80A of the container body 10A. Thus, the tear fluid is collected in the container body 10A. Due to the partition wall 20A in the container body 10A, the collected tear fluid does not easily flow into the irritant placement section 60A in which the eye irritant 90 is placed.

According to the above-described device 100A, tear fluid can be collected without directly pressing test paper against the subject's eyeball. The device 100A irritates the eye 1000 with the gas generated by the eye irritant 90 to stimulate tear fluid secretion. This enables efficient stimulation of tear fluid secretion. The device 100A is positioned such that the rim 15 of the container body 10A is in close contact with the face in the region around the eye. Accordingly, the gas generated by the eye irritant 90 is not easily dissipated into the surrounding space. Therefore, the eye 1000 can be efficiently irritated by the gas generated by the eye irritant 90. The tear fluid produced by secretion in the eye 1000 does not easily flow out of the container body 10A. Thus, the device 100A enables efficient tear fluid collection with less stress on the subject.

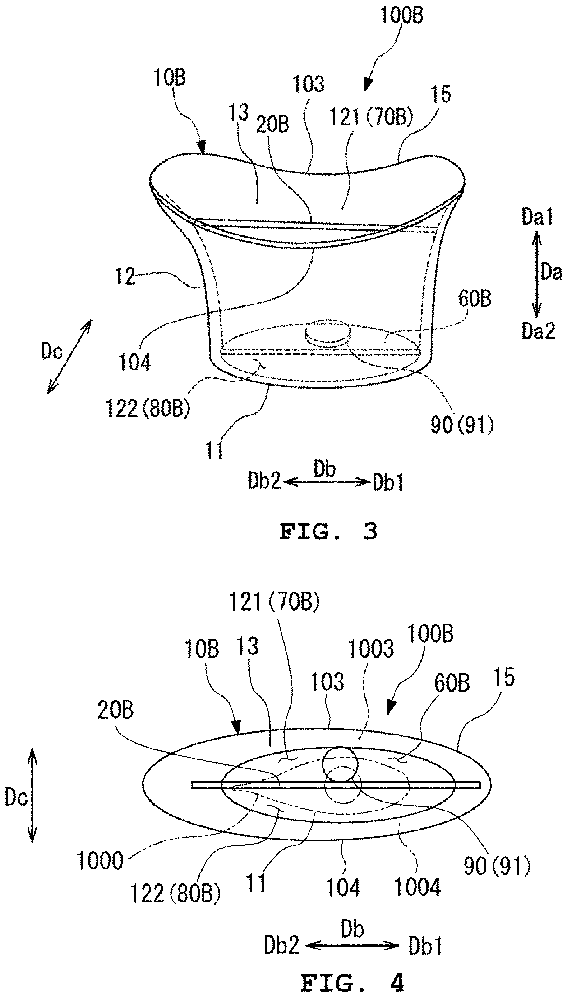

Figs. 3 and 4 illustrate a device 100B according to an embodiment. In the following description, structures that are the same as those in the device 100A illustrated in Figs. 1 and 2 are denoted by the same reference numerals as those in Figs. 1 and 2, and description thereof will be omitted.

The device 100B illustrated in Figs. 3 and 4 includes a container body 10B and a partition wall 20B.

The container body 10B includes the bottom portion 11, the peripheral wall 12, the opening 13, and the rim 15.

The partition wall 20B partitions the interior of the container body 10B into a plurality of spaces. The partition wall 20B is disposed between an upper end 103 and a lower end 104. The partition wall 20B is disposed at an intermediate location between the upper end 103 and the lower end 104 in the third direction Dc. The partition wall 20B may also be disposed closer to the upper end 103 or the lower end 104 than the intermediate location between the upper end 103 and the lower end 104 in the third direction Dc. The partition wall 20B extends along a plane substantially orthogonal to the third direction Dc. The partition wall 20B has an outer edge joined to the bottom portion 11 and the peripheral wall 12 of the container body 10B. The partition wall 20B partitions the interior of the container body 10B into an upper section 121 adjacent to the upper end 103 and a lower section 122 adjacent to the lower end 104.

A gas-receiving space 70B and a tear-fluid-receiving section 80B are provided in the container body 10B. The partition wall 20B partitions the interior of the container body 10B into the gas-receiving space 70B and the tear-fluid-receiving section 80B. In the present disclosure, the upper section 121 constitutes the gas-receiving space 70B. The lower section 122 constitutes the tear-fluid-receiving section 80B.

The gas-receiving space 70B is defined by the container body 10B. An irritant placement section 60B is provided in the container body 10B. The eye irritant 90 is disposed in the irritant placement section 60B. In the present disclosure, the eye irritant 90 is disposed in the gas-receiving space 70B. Accordingly, the irritant placement section 60B is disposed in at least a portion of the gas-receiving space 70B. Thus, the irritant placement section 60B communicates with the gas-receiving space 70B.

To collect tear fluid from the subject's eye 1000 by using the device 100B, the container body 10B is positioned such that the rim 15 is in close contact with the face in the region around the eye 1000. The upper end 103 is disposed adjacent to an upper eyelid 1003. The lower end 104 is disposed adjacent to a lower eyelid 1004.

The gas generated by the eye irritant 90 and present in the gas-receiving space 70B irritates the subject's eye 1000. As a result, tear fluid is produced by secretion in the eye 1000. The produced tear fluid flows out of the eye 1000. The tear fluid that has flowed out of the eye 1000 flows into the tear-fluid-receiving section 80B of the container body 10B. Thus, the tear fluid is collected in the container body 10B. Due to the partition wall 20B in the container body 10B, the collected tear fluid does not easily flow into the irritant placement section 60B in which the eye irritant 90 is placed.

The above-described device 100B enables efficient tear fluid collection with less stress on the subject.

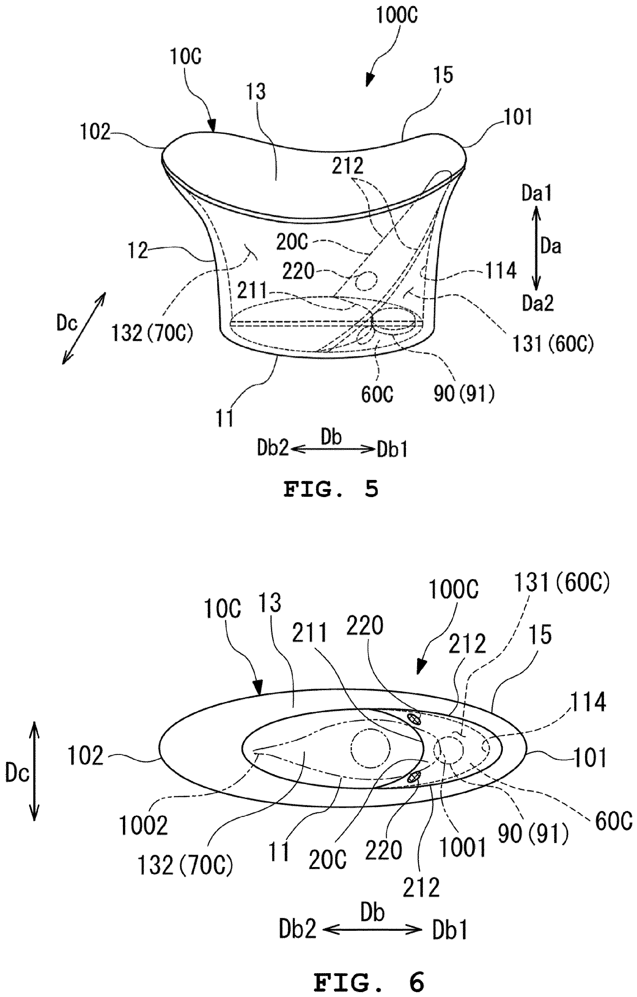

Figs. 5 and 6 illustrate a device 100C according to an embodiment. In the following description, structures that are the same as those in the device 100A illustrated in Figs. 1 and 2 are denoted by the same reference numerals as those in Figs. 1 and 2, and description thereof will be omitted.

The device 100C illustrated in Figs. 5 and 6 includes a container body 10C and a partition wall 20C.

The container body 10C includes the bottom portion 11, the peripheral wall 12, the opening 13, and the rim 15.

The partition wall 20C partitions the interior of the container body 10C into a plurality of spaces. The partition wall 20C is closer to the inner region of the container body 10C than the inner peripheral surface of the container body 10C. The entirety of an outer peripheral edge 21 of the partition wall 20C is joined to the container body 10C. The partition wall 20C has a bottom outer peripheral edge 211 that is curved in an arc shape when viewed in the first direction Da. The bottom outer peripheral edge 211 is joined to the bottom portion 11 at a position separated toward a second side Db2 in the second direction Db from an inner peripheral wall surface 114 of the peripheral wall 12 at the first end 101. The partition wall 20C has side outer peripheral edges 212 that are positioned at both sides of the partition wall 20C in a circumferential direction of the peripheral wall 12 and joined to the inner peripheral surface of the peripheral wall 12 at the first end 101. The partition wall 20C is shaped such that the width thereof in the circumferential direction gradually decreases in a direction from the other side Da2 toward the one side Da1 along the first direction Da. A sealed space 131 is formed between the partition wall 20C and the inner peripheral wall surface 114 of the peripheral wall 12 at the first end 101. The partition wall 20C partitions the interior of the container body 10C into the sealed space 131 and an open space 132 other than the sealed space 131. The open space 132 opens toward the one side Da1 in the first direction Da at the opening 13.

The partition wall 20C has ventilation holes 220 through which regions on both sides of the partition wall 20C communicate with each other. For example, the partition wall 20C has two ventilation holes 220 at both sides thereof in the circumferential direction of the peripheral wall 12. The number of ventilation holes 220 is not limited to two. The number of ventilation holes 220 may be one or three or more. The positions of the ventilation holes 220 are not limited to specific positions as long as the ventilation holes 220 provide communication with a gas-receiving space 70C in the container body 10C.

An irritant placement section 60C is provided in the container body 10C. The eye irritant 90 is disposed in the irritant placement section 60C. The irritant placement section 60C is formed in the sealed space 131.

The gas-receiving space 70C is defined in the container body 10C. In the embodiment of the present disclosure, the open space 132 constitutes the gas-receiving space 70C. The gas generated by the eye irritant 90 disposed in the irritant placement section 60C in the sealed space 131 is supplied to the gas-receiving space 70C through the ventilation holes 220.

To collect tear fluid from the subject's eye 1000 by using the device 100C, the container body 10C is positioned such that the rim 15 is in close contact with the face in the region around the eye 1000. The subject tilts their head so that the outer corner 1002 of the eye 1000 against which the container body 10C is pressed is lowered with respect to the inner corner 1001.

The gas generated by the eye irritant 90 is supplied to the gas-receiving space 70C, so that the subject's eye 1000 is irritated. As a result, tear fluid is produced by secretion in the eye 1000 and flows out of the eye 1000 from the outer corner 1002 at a lower position. The tear fluid that has flowed out of the eye 1000 flows into a tear-fluid-receiving section 80C of the container body 10C. Thus, the tear fluid is collected in the container body 10C. Due to the partition wall 20C in the container body 10C, the collected tear fluid does not easily flow into the irritant placement section 60C in which the eye irritant 90 is placed.

The above-described device 100C enables efficient tear fluid collection with less stress on the subject.

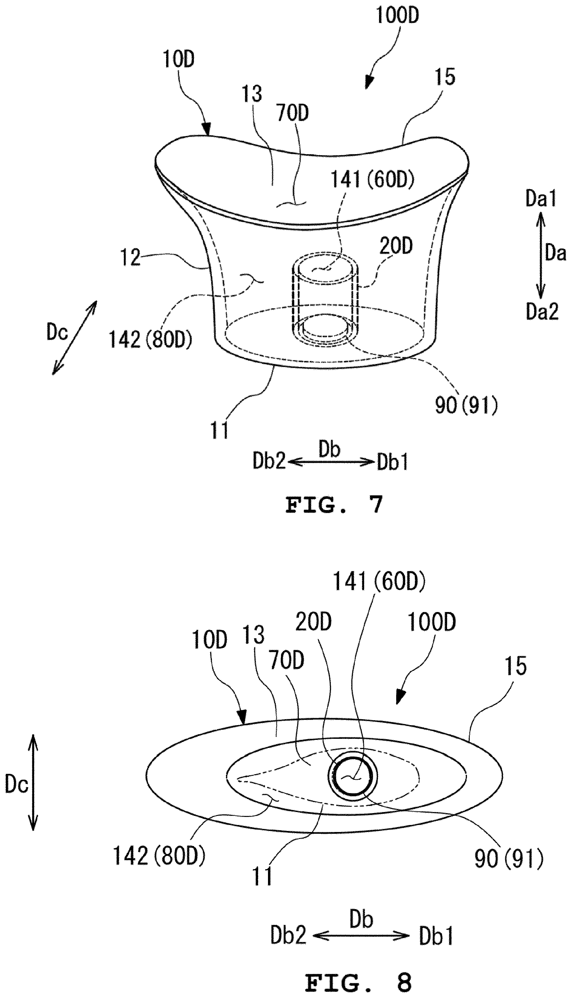

Figs. 7 and 8 illustrate a device 100D according to an embodiment. In the following description, structures that are the same as those in the device 100A illustrated in Figs. 1 and 2 are denoted by the same reference numerals as those in Figs. 1 and 2, and description thereof will be omitted.

The device 100D illustrated in Figs. 7 and 8 includes a container body 10D and a partition wall 20D.

The container body 10D includes the bottom portion 11, the peripheral wall 12, the opening 13, and the rim 15.

The partition wall 20D partitions the interior of the container body 10D into a plurality of spaces. The partition wall 20D is formed on the bottom portion 11. The partition wall 20D has a tubular shape. The partition wall 20D extends from the bottom portion 11 toward the one side Da1 in the first direction Da. The height of the partition wall 20D from the bottom portion 11 is less than that of the rim 15 of the container body 10D. The partition wall 20D has a circular cross section when viewed in the first direction Da. The cross sectional shape of the partition wall 20D may also be, for example, an elliptical shape or a polygonal shape. The partition wall 20D is disposed in a central region of the bottom portion 11 when viewed in the first direction Da. The partition wall 20D partitions the interior of the container body 10D into an inner section 141 and an outer section 142. The inner section 141 is formed on the radially inner side of the partition wall 20D. The outer section 142 is formed on the radially outer side of the partition wall 20D.

A gas-receiving space 70D and a tear-fluid-receiving section 80D are provided in the container body 10D. The partition wall 20D partitions the interior of the container body 10D into an irritant placement section 60D and the tear-fluid-receiving section 80D. In the present disclosure, the inner section 141 constitutes the irritant placement section 60D. The outer section 142 constitutes the tear-fluid-receiving section 80D.

The gas-receiving space 70D is defined by the container body 10D. The entire space inside the container body 10D can be filled with the gas generated by the eye irritant 90 disposed in the irritant placement section 60D. In the embodiment of the present disclosure, the entire space inside the container body 10D constitutes the gas-receiving space 70D.

To collect tear fluid from the subject's eye 1000 by using the device 100D, the container body 10D is positioned such that the rim 15 is in close contact with the face in the region around the eye 1000.

The gas generated by the eye irritant 90 and present in the gas-receiving space 70D irritates the subject's eye 1000. As a result, tear fluid is produced by secretion in the eye 1000. The produced tear fluid flows out of the eye 1000. The tear fluid that has flowed out of the eye 1000 flows into the tear-fluid-receiving section 80D of the container body 10D. Thus, the tear fluid is collected in the container body 10D. Due to the partition wall 20D in the container body 10D, the collected tear fluid does not easily flow into the irritant placement section 60D in which the eye irritant 90 is placed.

The above-described device 100D enables efficient tear fluid collection with less stress on the subject.

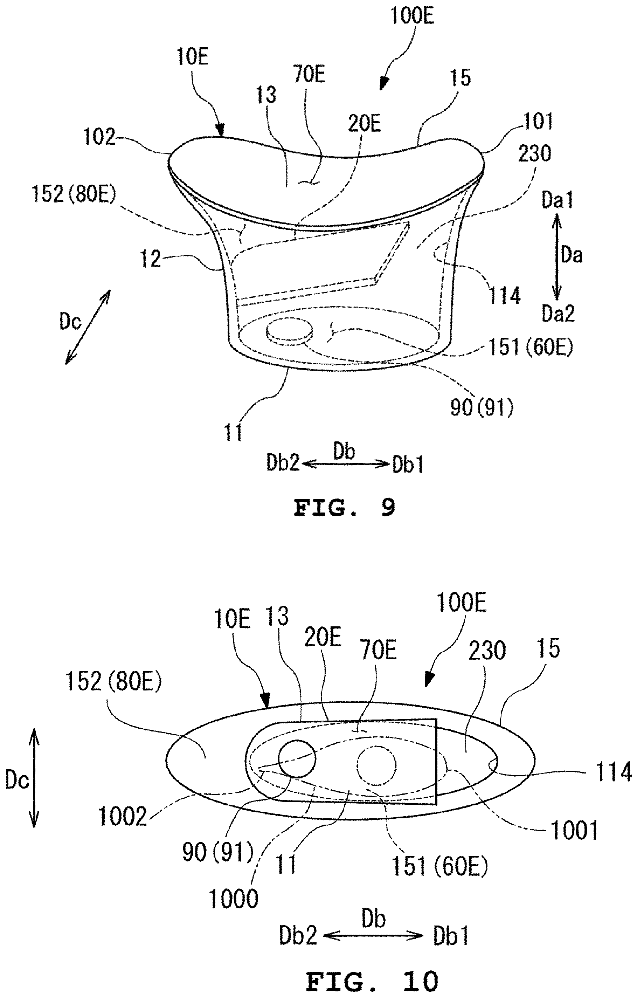

Figs. 9 and 10 illustrate a device 100E according to an embodiment. In the following description, structures that are the same as those in the device 100A illustrated in Figs. 1 and 2 are denoted by the same reference numerals as those in Figs. 1 and 2, and description thereof will be omitted.

The device 100E illustrated in Figs. 9 and 10 includes a container body 10E and a partition wall 20E.

The container body 10E includes the bottom portion 11, the peripheral wall 12, the opening 13, and the rim 15.

The partition wall 20E partitions the interior of the container body 10E into a plurality of spaces. The partition wall 20E is disposed on the peripheral wall 12 at an intermediate position in the first direction Da. The partition wall 20E is separated from the bottom portion 11 toward the one side Da1 in the first direction Da. The partition wall 20E extends along a plane that crosses the first direction Da. An end of the partition wall 20E at the first side Db1 in the second direction Db is spaced from the inner peripheral wall surface 114 at the first end 101 with a communicating portion 230 provided therebetween.

The partition wall 20E is inclined with respect to a plane orthogonal to the first direction Da toward the other side Da2 in the first direction Da in a direction from the first end 101 toward the second end 102 of the container body 10E. The partition wall 20E partially blocks the inner space of the container body 10E when viewed in the first direction Da.

The partition wall 20E partially partitions the space inside the container body 10E into a bottom-portion-side space 151 and an opening-side space 152. In the container body 10E, the bottom-portion-side space 151 is formed on the other side Da2 (side adjacent to the bottom portion 11) of the partition wall 20E in the first direction Da. The opening-side space 152 is on the one side Da1 (side adjacent to the opening 13) of the partition wall 20E in the first direction Da. The bottom-portion-side space 151 and the opening-side space 152 communicate with each other through the communicating portion 230.

An irritant placement section 60E and a tear-fluid-receiving section 80E are provided in the container body 10E. The partition wall 20E partially partitions the interior of the container body 10E into the irritant placement section 60E and the tear-fluid-receiving section 80E. In the present disclosure, the bottom-portion-side space 151 constitutes the irritant placement section 60E. The opening-side space 152 constitutes the tear-fluid-receiving section 80E.

A gas-receiving space 70E is defined by the container body 10E. The gas generated by the eye irritant 90 disposed in the irritant placement section 60E flows into the opening-side space 152 from the bottom-portion-side space 151 through the communicating portion 230. The entire space inside the container body 10E can be filled with the gas generated by the eye irritant 90. In the embodiment of the present disclosure, the entire space inside the container body 10E constitutes the gas-receiving space 70E.

To collect tear fluid from the subject's eye 1000 by using the device 100E, the container body 10E is positioned such that the rim 15 is in close contact with the face in the region around the eye 1000. The subject tilts their head so that the outer corner 1002 of the eye 1000 against which the container body 10E is pressed is lowered with respect to the inner corner 1001.

The gas generated by the eye irritant 90 and present in the gas-receiving space 70E irritates the subject's eye 1000. As a result, tear fluid is produced by secretion in the eye 1000. The produced tear fluid flows out of the eye 1000. The tear fluid that has flowed out of the eye 1000 flows into the tear-fluid-receiving section 80E of the container body 10E. Thus, the tear fluid is collected in the container body 10E. Due to the partition wall 20E in the container body 10E, the collected tear fluid does not easily flow into the irritant placement section 60E in which the eye irritant 90 is placed. The partition wall 20E is inclined with respect to a plane orthogonal to the first direction Da toward the other side Da2 in the direction from the first end 101 toward the second end 102 of the container body 10E. Therefore, the tear fluid does not easily flow toward the communicating portion 230.

The above-described device 100E enables efficient tear fluid collection with less stress on the subject.

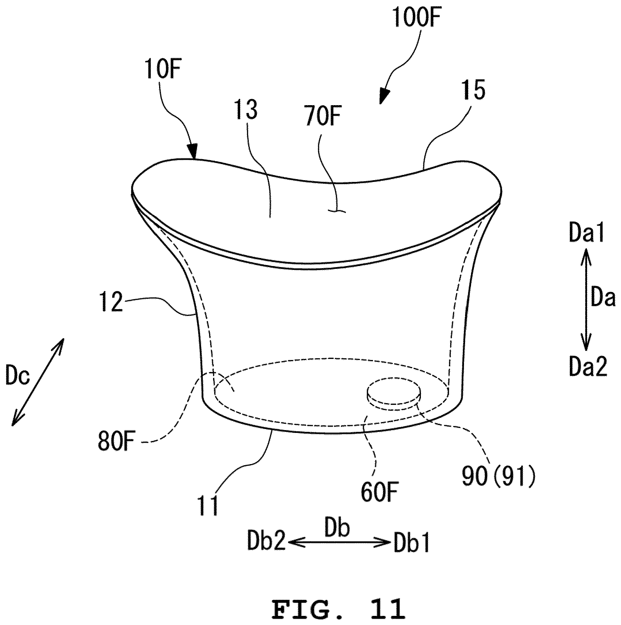

Fig. 11 illustrates a device 100F according to an embodiment. In the following description, structures that are the same as those in the device 100A illustrated in Figs. 1 and 2 are denoted by the same reference numerals as those in Figs. 1 and 2, and description thereof will be omitted.

The device 100F illustrated in Fig. 11 includes a container body 10F. The container body 10F includes the bottom portion 11, the peripheral wall 12, the opening 13, and the rim 15. An irritant placement section 60F, a gas-receiving space 70F, and a tear-fluid-receiving section 80F are provided in the container body 10F.

The irritant placement section 60F is disposed on the bottom portion 11. The irritant placement section 60F may be disposed on the bottom portion 11 at a position shifted toward the first side Db1 in the second direction Db. The irritant placement section 60F may be such that, for example, the eye irritant 90 is simply placed on the bottom portion 11. The irritant placement section 60F may also be such that, for example, the eye irritant 90 is fixed to the bottom portion 11 with an adhesive or the like.

The gas-receiving space 70F is defined by the container body 10F. The entire space inside the container body 10F can be filled with the gas generated by the eye irritant 90 disposed in the irritant placement section 60F. In the embodiment of the present disclosure, the entire space inside the container body 10F constitutes the gas-receiving space 70F.

The tear-fluid-receiving section 80F is provided in the container body 10F. To collect tear fluid from the subject's eye by using the device 100F, the container body 10F is positioned such that the rim 15 is in close contact with the face in the region around the eye 1000. The subject tilts their head so that the outer corner 1002 of the eye 1000 against which the container body 10F is pressed is lowered with respect to the inner corner 1001. The tear-fluid-receiving section 80F is provided in the container body 10F in a region adjacent to the outer corner 1002 at the second side Db2 in the second direction Db.

The gas generated by the eye irritant 90 is supplied to the gas-receiving space 70F, so that the subject's eye 1000 is irritated. As a result, tear fluid is produced by secretion in the eye 1000 and flows out of the eye 1000 from the outer corner 1002 at a lower position. The tear fluid that has flowed out of the eye 1000 flows into the tear-fluid-receiving section 80F of the container body 10F. Thus, the tear fluid is collected in the container body 10F.

The above-described device 100F enables efficient tear fluid collection with less stress on the subject.

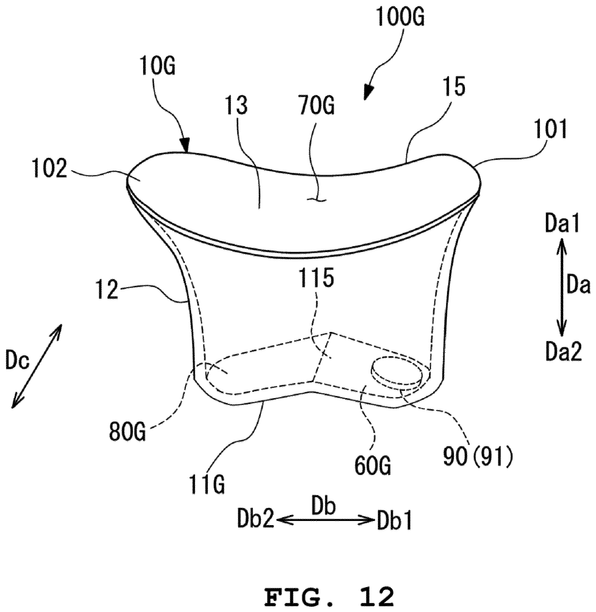

Fig. 12 illustrates a device 100G according to an embodiment. In the following description, structures that are the same as those in the device 100A illustrated in Figs. 1 and 2 are denoted by the same reference numerals as those in Figs. 1 and 2, and description thereof will be omitted.

The device 100G illustrated in Fig. 12 includes a container body 10G. The container body 10G includes a bottom portion 11G, the peripheral wall 12, the opening 13, and the rim 15.

The bottom portion 11G includes a bottom intermediate section 115 that bulges between the first end 101 and the second end 102 in the second direction Db. The bottom portion 11G is inclined toward the other side Da2 (toward the bottom portion 11) in the first direction Da from the bottom intermediate section 115 toward the first end 101 at the one side Da1 in the second direction Db and toward the second end 102 at the other side Da2 in the second direction Db.

An irritant placement section 60G, a gas-receiving space 70G, and a tear-fluid-receiving section 80G are provided in the container body 10G.

The irritant placement section 60G is disposed on the bottom portion 11G. The irritant placement section 60G may be disposed on the bottom portion 11G at a location on the first side Db1 of the bulging bottom intermediate section 115 in the second direction Db.

The gas-receiving space 70G is defined by the container body 10G. The entire space inside the container body 10G can be filled with the gas generated by the eye irritant 90 disposed in the irritant placement section 60G. In the embodiment of the present disclosure, the entire space inside the container body 10G constitutes the gas-receiving space 70G.

The tear-fluid-receiving section 80G is provided in the container body 10G. to collect tear fluid from the subject's eye 1000 by using the device 100G, the container body 10G is positioned such that the rim 15 is in close contact with the face in the region around the eye 1000. The subject tilts their head so that the outer corner 1002 of the eye 1000 against which the container body 10G is pressed is lowered with respect to the inner corner 1001. The tear-fluid-receiving section 80G is provided in the container body 10G in a region on the second side Db2 of the bulging bottom intermediate section 115 in the second direction Db.

The gas generated by the eye irritant 90 is supplied to the gas-receiving space 70G, so that the subject's eye 1000 is irritated. As a result, tear fluid is produced by secretion in the eye 1000 and flows out of the eye 1000 from the outer corner 1002 at a lower position. The tear fluid that has flowed out of the eye 1000 flows into the tear-fluid-receiving section 80G of the container body 10G. Thus, the tear fluid is collected in the container body 10G. The above-described device 100G enables efficient tear fluid collection with less stress on the subject.

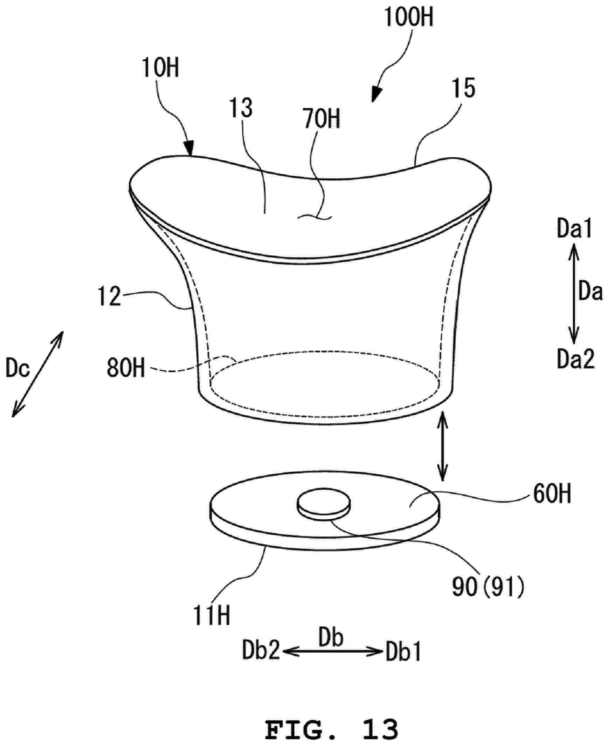

Fig. 13 illustrates a device 100H according to an embodiment. In the following description, structures that are the same as those in the device 100A illustrated in Figs. 1 and 2 are denoted by the same reference numerals as those in Figs. 1 and 2, and description thereof will be omitted.

The device 100H illustrated in Fig. 13 includes a container body 10H. The container body 10H includes a bottom portion 11H, the peripheral wall 12, the opening 13, and the rim 15. An irritant placement section 60H, a gas-receiving space 70H, and a tear-fluid-receiving section 80H are provided in the container body 10H.

The bottom portion 11H is removably attachable to the peripheral wall 12. The bottom portion 11H may also be openable and closable with respect to the peripheral wall 12. The irritant placement section 60H is disposed on the bottom portion 11H. The irritant placement section 60H may be such that, for example, the eye irritant 90 is simply placed on the bottom portion 11H. The irritant placement section 60H may be such that, for example, the eye irritant 90 is fixed to the bottom portion 11H with an adhesive or the like.

The gas-receiving space 70H is defined by the container body 10H. The entire space inside the container body 10H can be filled with the gas generated by the eye irritant 90 disposed in the irritant placement section 60H. In the embodiment of the present disclosure, the entire space inside the container body 10H constitutes the gas-receiving space 70H.

The tear-fluid-receiving section 80H is provided in the container body 10H. to collect tear fluid from the subject's eye 1000 by using the device 100H, the container body 10H is positioned such that the rim 15 is in close contact with the face in the region around the eye 1000. The subject tilts their head so that the outer corner 1002 of the eye 1000 against which the container body 10H is pressed is lowered with respect to the inner corner 1001. The tear-fluid-receiving section 80H is provided in the container body 10H in a region adjacent to the outer corner 1002 at the second side Db2 in the second direction Db.

To collect tear fluid from the subject's eye 1000 by using the device 100H, first, the container body 10H is positioned such that the rim 15 is in close contact with the face in the region around the eye 1000. At this time, the bottom portion 11H may be separated from the peripheral wall 12. After the device 100H is placed on the subject, the bottom portion 11H on which the eye irritant 90 is mounted is attached to the peripheral wall 12. The eye irritant 90 is set to a state in which gas can be generated therefrom by dripping a substrate solution or water onto the eye irritant 90.

The gas generated by the eye irritant 90 is supplied to the gas-receiving space 70H, so that the subject's eye 1000 is irritated. As a result, tear fluid is produced by secretion in the eye 1000 and flows out of the eye 1000 from the outer corner 1002 at a lower position. The tear fluid that has flowed out of the eye 1000 flows into the tear-fluid-receiving section 80H of the container body 10H. Thus, the tear fluid is collected in the container body 10H. The above-described device 100H enables efficient tear fluid collection with less stress on the subject.

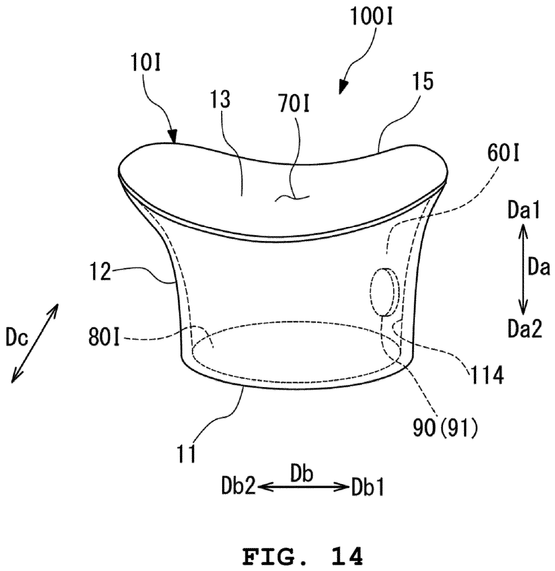

Fig. 14 illustrates a device 100I according to an embodiment. In the following description, structures that are the same as those in the device 100A illustrated in Figs. 1 and 2 are denoted by the same reference numerals as those in Figs. 1 and 2, and description thereof will be omitted.

The device 100I illustrated in Fig. 14 includes a container body 10I. The container body 10I includes the bottom portion 11, the peripheral wall 12, the opening 13, and the rim 15. An irritant placement section 60I, a gas-receiving space 70I, and a tear-fluid-receiving section 80I are provided in the container body 10I.

The irritant placement section 60I is disposed on an inner peripheral surface of the peripheral wall 12. The irritant placement section 60I may be disposed on the inner peripheral surface of the peripheral wall 12 at a position shifted toward the first side Db1 in the second direction Db. The irritant placement section 60I is provided on the inner peripheral wall surface 114 of the peripheral wall 12 at the first end 101. The irritant placement section 60I may be such that, for example, the eye irritant 90 is fixed to the inner peripheral wall surface 114 with an adhesive or the like.

The gas-receiving space 70I is defined by the container body 10I. The entire space inside the container body 10I can be filled with the gas generated by the eye irritant 90 disposed in the irritant placement section 60I. In the embodiment of the present disclosure, the entire space inside the container body 10I constitutes the gas-receiving space 70I.

The tear-fluid-receiving section 80I is provided in the container body 10I. to collect tear fluid from the subject's eye 1000 by using the device 100I, the container body 10I is positioned such that the rim 15 is in close contact with the face in the region around the eye 1000. The subject tilts their head so that the outer corner 1002 of the eye 1000 against which the container body 10I is pressed is lowered with respect to the inner corner 1001. The tear-fluid-receiving section 80I is provided in the container body 10I in a region adjacent to the outer corner 1002 at the second side Db2 in the second direction Db.

The gas generated by the eye irritant 90 is supplied to the gas-receiving space 70I, so that the subject's eye 1000 is irritated. As a result, tear fluid is produced by secretion in the eye 1000 and flows out of the eye 1000 from the outer corner 1002 at a lower position. The tear fluid that has flowed out of the eye 1000 flows into the tear-fluid-receiving section 80I of the container body 10I. Thus, the tear fluid is collected in the container body 10I. The above-described device 100I enables efficient tear fluid collection with less stress on the subject.

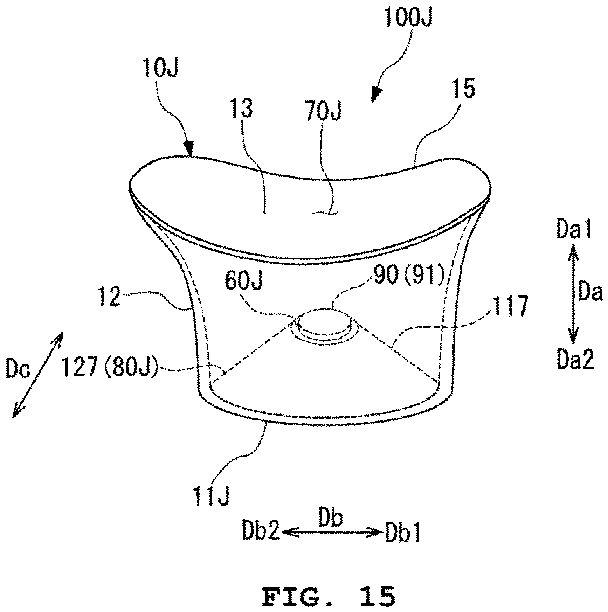

Fig. 15 illustrates a device 100J according to an embodiment. In the following description, structures that are the same as those in the device 100A illustrated in Figs. 1 and 2 are denoted by the same reference numerals as those in Figs. 1 and 2, and description thereof will be omitted.

The device 100J illustrated in Fig. 15 includes a container body 10J. The container body 10J includes a bottom portion 11J, the peripheral wall 12, the opening 13, and the rim 15.

The bottom portion 11J includes a base 117. The base 117 bulges toward the one side Da1 in the first direction Da from the peripheral edge of the bottom portion 11J toward the inner region of the bottom portion 11J. The base 117 has a circular shape when viewed in the first direction Da. The base 117 is shaped such that the outer diameter thereof gradually decreases from the bottom portion 11J toward the one side Da1 in the first direction Da. The base 117 substantially has a truncated cone shape. The bottom portion 11J has an annular recess 127 in a region radially outside the base 117. The annular recess 127 has a circular annular shape when viewed in the first direction Da.

An irritant placement section 60J, a gas-receiving space 70J, and a tear-fluid-receiving section 80J are provided in the container body 10J.

The irritant placement section 60J is disposed on the base 117. The gas-receiving space 70J is defined by the container body 10J. The entire space inside the container body 10J can be filled with the gas generated by the eye irritant 90 disposed in the irritant placement section 60J. In the embodiment of the present disclosure, the entire space inside the container body 10J constitutes the gas-receiving space 70J.

The tear-fluid-receiving section 80J is provided in the container body 10J. The tear-fluid-receiving section 80J is provided in the annular recess 127 formed in the bottom portion 11J.

The gas generated by the eye irritant 90 is supplied to the gas-receiving space 70J, so that the subject's eye 1000 is irritated. As a result, tear fluid is produced by secretion in the eye 1000 and flows out of the eye 1000 from the outer corner 1002 at a lower position. The tear fluid that has flowed out of the eye 1000 flows into the tear-fluid-receiving section 80J of the container body 10J. Thus, the tear fluid is collected in the container body 10J. The above-described device 100J enables efficient tear fluid collection with less stress on the subject.

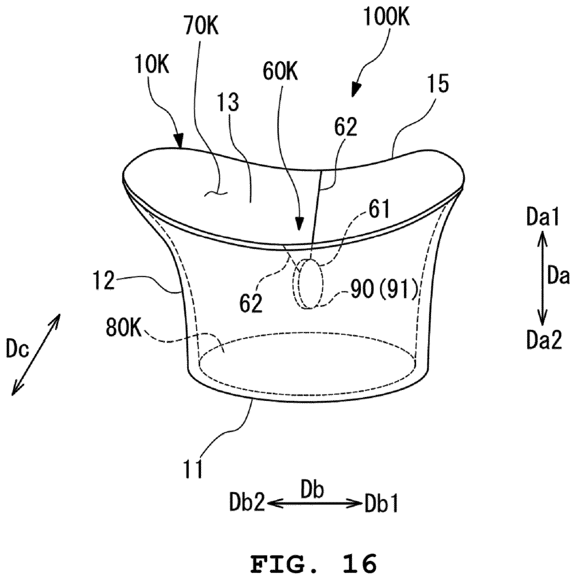

Fig. 16 illustrates a device 100K according to an embodiment. In the following description, structures that are the same as those in the device 100A illustrated in Figs. 1 and 2 are denoted by the same reference numerals as those in Figs. 1 and 2, and description thereof will be omitted.

The device 100K illustrated in Fig. 16 includes a container body 10K.

The container body 10K includes the bottom portion 11, the peripheral wall 12, the opening 13, and the rim 15. An irritant placement section 60K, a gas-receiving space 70K, and a tear-fluid-receiving section 80K are provided in the container body 10K.

The irritant placement section 60K includes a holder body 61 and connection members 62. The holder body 61 holds the eye irritant 90. The holder body 61 may, for example, have a frame shape that corresponds to the outer shape of the eye irritant 90 to be capable of holding an outer peripheral portion of the eye irritant 90. The holder body 61 may have any other structure as long as the holder body 61 is capable of holding the eye irritant 90. One end of each connection member 62 is connected to the holder body 61. The other end of each connection member 62 is connected to the container body 10K. The connection members 62 are provided on both sides of the holder body 61. The eye irritant 90 held by the holder body 61 is retained by the connection members 62. In the embodiment of the present disclosure, two connection members 62, for example, are provided. The number of connection members 62 may be three or more. The holder body 61 supported by the connection members 62 and the eye irritant 90 may be spaced from the bottom portion 11 toward the one side Da1 of the first direction Da. The holder body 61 supported by the connection members 62 and the eye irritant 90 may be in contact with the bottom portion 11. Since the connection members 62 are suspended in the inner space of the container body 10K, for example, the efficiency of vaporization of the lachrymatory component is increased.

The gas-receiving space 70K is defined by the container body 10K. The entire space inside the container body 10K can be filled with the gas generated by the eye irritant 90 disposed in the irritant placement section 60K. In the embodiment of the present disclosure, the entire space inside the container body 10K constitutes the gas-receiving space 70K.

The tear-fluid-receiving section 80K is provided in the container body 10K. The gas generated by the eye irritant 90 is supplied to the gas-receiving space 70K, so that the subject's eye 1000 is irritated. As a result, tear fluid is produced by secretion in the eye 1000 and flows into the tear-fluid-receiving section 80K of the container body 10K. When the holder body 61 supported by the connection members 62 and the eye irritant 90 are spaced from the bottom portion 11 toward the one side Da1 in the first direction Da, the tear fluid does not easily come into contact with the eye irritant 90. The above-described device 100K enables efficient tear fluid collection with less stress on the subject.

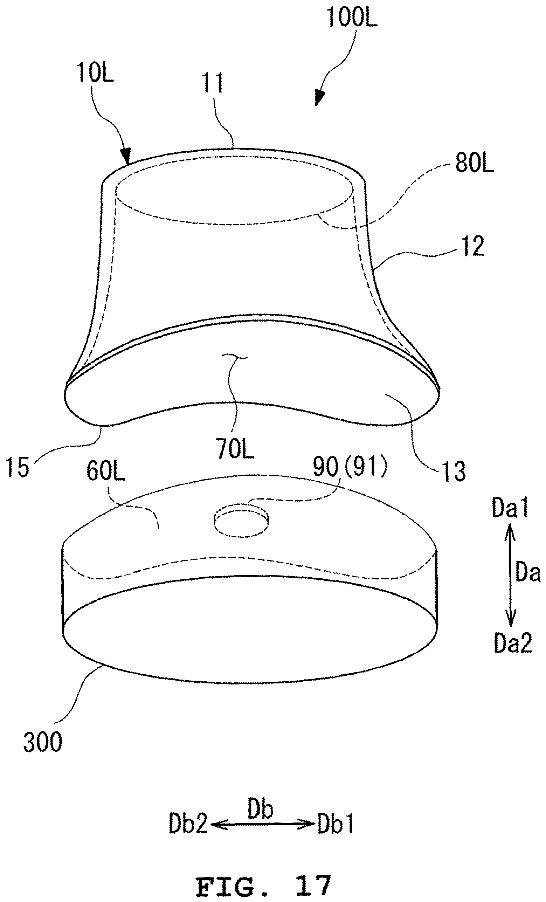

Fig. 17 illustrates a device 100L according to an embodiment. In the following description, structures that are the same as those in the device 100A illustrated in Figs. 1 and 2 are denoted by the same reference numerals as those in Figs. 1 and 2, and description thereof will be omitted.

The device 100L illustrated in Fig. 17 includes a container body 10L and a removably attachable member 300.

The container body 10L includes the bottom portion 11, the peripheral wall 12, the opening 13, and the rim 15.

The removably attachable member 300 is removably attachable to the opening 13. The removably attachable member 300 is configured to close the opening 13 when attached to the opening 13.

An irritant placement section 60L is disposed on the removably attachable member 300. When the removably attachable member 300 is attached to the opening 13, the irritant placement section 60L is disposed on a surface of the removably attachable member 300 that faces the container body 10L.

The irritant placement section 60L holds the eye irritant 90. The irritant placement section 60L is attached to the opening 13 of the container body 10L after a substrate solution or water is dripped onto the eye irritant 90 so that gas can be generated by the eye irritant 90. Thus, the gas is supplied to the inner region of the container body 10L.

A gas-receiving space 70L is defined by the container body 10L. The entire space inside the container body 10L can be filled with the gas generated by the eye irritant 90 disposed in the irritant placement section 60L. In the embodiment of the present disclosure, the entire space inside the container body 10L constitutes the gas-receiving space 70L. A tear-fluid-receiving section 80L is provided in the container body 10L.

To collect tear fluid, the removably attachable member 300 is attached to the opening 13 of the container body 10L in a state such that gas can be generated by the eye irritant 90. The removably attachable member 300 is removed from the opening 13 after the gas generated by the eye irritant 90 is supplied to the gas-receiving space 70L. The user brings the rim 15 of the container body 10L into close contact with their face in the region around the eye 1000 before the gas dissipates from the gas-receiving space 70L. Thus, the subject's eye 1000 is irritated by the gas. As a result, tear fluid is produced by secretion in the eye 1000 and flows into the tear-fluid-receiving section 80L of the container body 10L. The above-described device 100L enables efficient tear fluid collection with less stress on the subject.

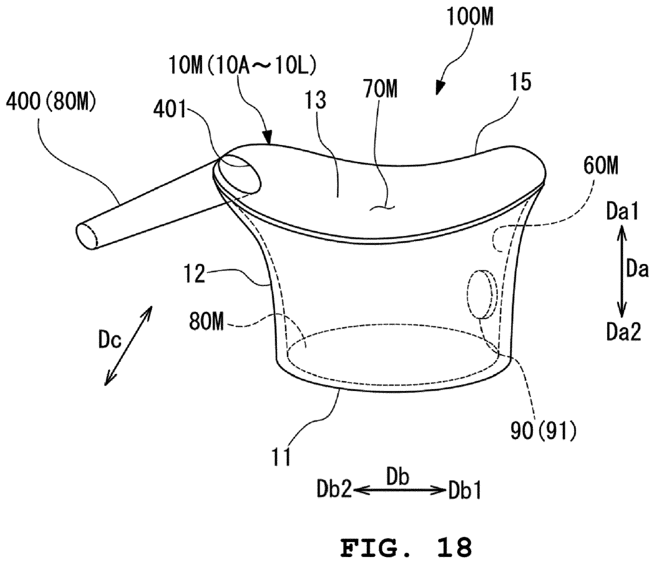

Fig. 18 illustrates a device 100M according to an embodiment. In the following description, structures that are the same as those in the device 100A illustrated in Figs. 1 and 2 are denoted by the same reference numerals as those in Figs. 1 and 2, and description thereof will be omitted.

The device 100M illustrated in Fig. 14 includes a container body 10M and a tear fluid container 400.

The container body 10M may be one of the above-described container bodies 10A to 10L.

The tear fluid container 400 is provided integrally with the container body 10M. The tear fluid received by the tear-fluid-receiving section 80M is collected in the tear fluid container 400. The tear fluid container 400 is disposed to extend outward from the opening 13 of the container body 10M. The tear fluid container 400 has a tubular shape with a bottom. The tear fluid container 400 has a container opening 401 that opens toward the inner corner 1001 when the rim 15 of the container body 10M is in close contact with the face in a region around the eye 1000.

The subject tilts their head so that the outer corner 1002 of the eye 1000 against which the container body 10M is pressed is lowered with respect to the inner corner 1001. The gas generated by the eye irritant 90 disposed in the irritant placement section 60M is supplied to the gas-receiving space 70M, so that the subject's eye 1000 is irritated. As a result, tear fluid is produced by secretion in the eye 1000 and flows out of the eye 1000 from the outer corner 1002 at a lower position. The tear fluid that has flowed out of the eye 1000 flows into the tear-fluid-receiving section 80M of the container body 10M. The tear fluid in the tear-fluid-receiving section 80M of the container body 10M can be collected in the tear fluid container 400 by tilting the device 100M so as to lower the tear fluid container 400. The tear fluid that has flowed out of the eye 1000 may be directly collected in the tear fluid container 400. In this case, the tear fluid container 400 constitutes the tear-fluid-receiving section 80M.

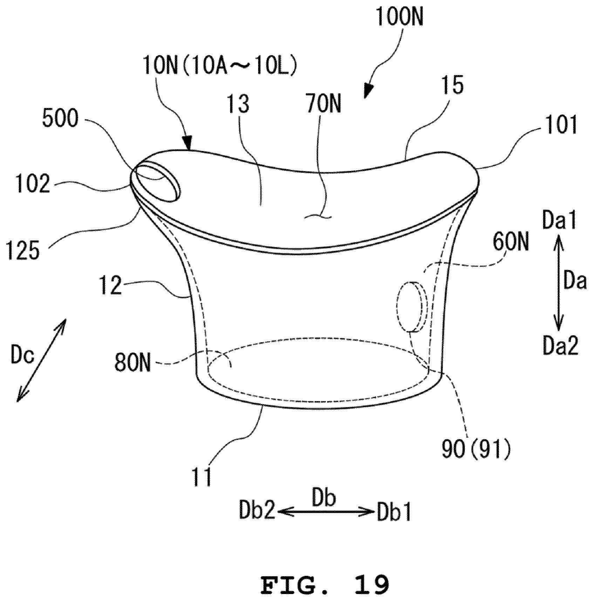

Fig. 19 illustrates a device 100N according to an embodiment. In the following description, structures that are the same as those in the device 100A illustrated in Figs. 1 and 2 are denoted by the same reference numerals as those in Figs. 1 and 2, and description thereof will be omitted.

The device 100N illustrated in Fig. 19 includes a container body 10N and has an access opening 500. The container body 10N may be one of the above-described container bodies 10A to 10L.

The access opening 500 is formed, for example, at the peripheral edge 125 of the container body 10N. The access opening 500 is formed to enable access to the inside of the container body 10N from the outside when the rim 15 is in close contact with the face in the region around the eye 1000. The access opening 500 is used to collect the tear fluid received by a tear-fluid-receiving section 80N of the container body 10N with a collecting tool, such as a dropper. The tear fluid in the tear-fluid-receiving section 80N is accessible by the collecting tool through the access opening 500. The access opening 500 may also be used to introduce the gas generated by the eye irritant 90 disposed outside the container body 10N into the container body 10N. In some embodiments, the access opening 500 may be substantially sealable. It is not necessary that the access opening be completely sealed. For example, the access opening may be sealed to an extent such that a sufficiently large lachrymatory effect can be exerted on the eyeball by the lachrymatory component. For example, the accelerator opening may be provided with an openable and closable lid. For example, the accelerator opening may be provided with an elastic lid with radial slits (such as a garbage disposal splash guard).

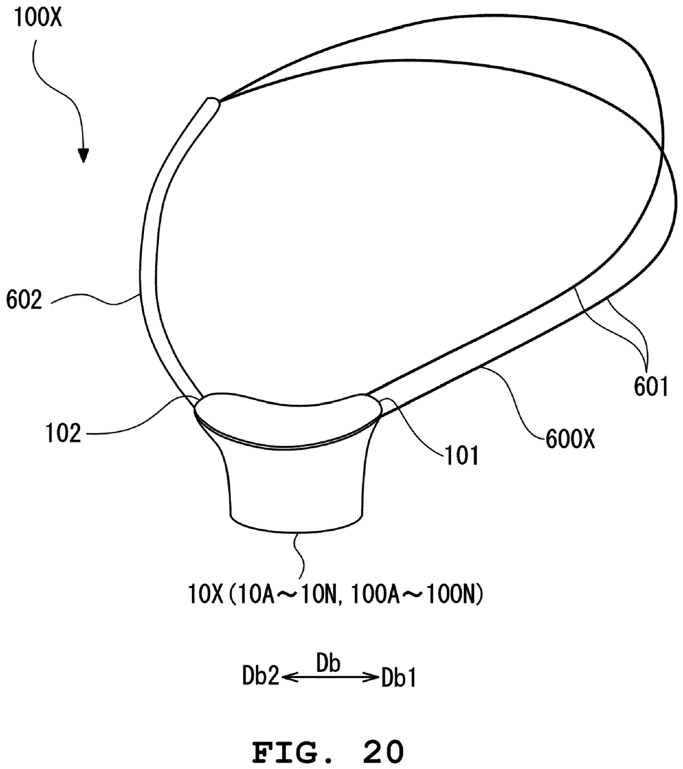

Fig. 20 illustrates a device 100X according to an embodiment. The device 100X illustrated in Fig. 20 includes a container body 10X and a retainer 600X.

The container body 10X may be one of the above-described container bodies 10A to 10N (devices 100A to 100N) .

The retainer 600X retains the container body 10X on the head of the user so that the rim 15 of the container body 10X is in close contact with the face in the region around the eye 1000. The retainer 600X may include, for example, a rubber string (rubber band), a string, a belt, or tape with a hook-and-loop fastener. For example, two strings 601 may be arranged such that one end of each string 601 is connected to one end of the container body 10X in the second direction Db. One end of a rubber string 602 is connected to the other end of the container body 10X in the second direction Db. The other end of each of the two strings 601 is connected to the other end of the rubber string 602. The container body 10X is retained on the subject's head by the retainer 600X.

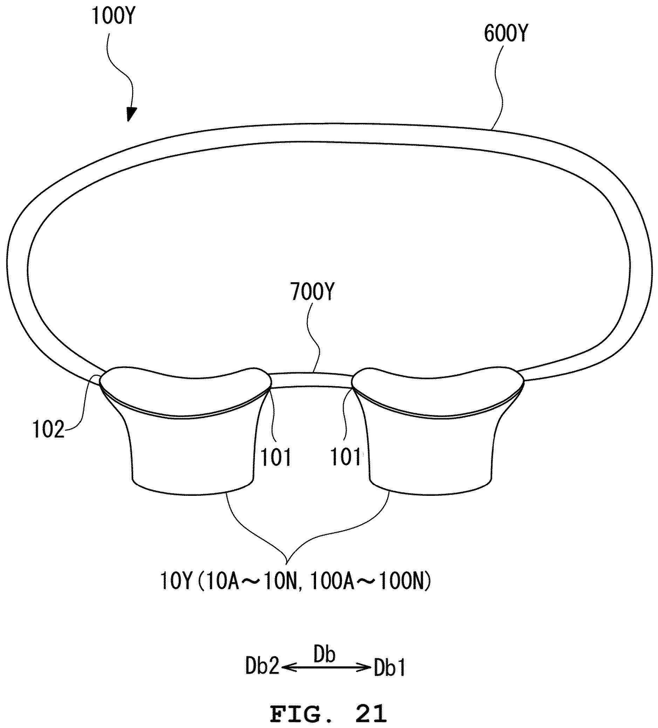

Fig. 21 illustrates a device 100Y according to an embodiment. The device 100Y illustrated in Fig. 21 includes two container bodies 10Y, a connector 700Y, and a retainer 600Y.

Each container body 10Y may be one of the above-described container bodies 10A to 10N (devices 100A to 100N) .

The connector 700Y connects the first ends 101 of the two container bodies 10Y to each other. The first ends 101 of the container bodies 10Y are inner-corner end portions of the container bodies 10Y that are adjacent to the inner corners 1001 when the rims 15 of the two container bodies 10Y are in close contact with the face in the regions around the eyes 1000.

The retainer 600Y retains the two container bodies 10Y on the head of the user so that the rims 15 of the container bodies 10Y are in close contact with the face in the regions around the eyes 1000. The retainer 600Y may include, for example, a rubber string (rubber band), a string, a belt, or tape with a hook-and-loop fastener. The retainer 600Y connects the second ends 102 of the two container bodies 10Y to each other. The second ends 102 are outer-corner end portions that are adjacent to the outer corners 1002 when the rims 15 of the two container bodies 10Y are in close contact with the face in the regions around the eyes 1000. to put the device 100Y on the user, the retainer 600Y is placed on the back of the user's head while the rims 15 of the two container bodies 10Y are in close contact with the face in the regions around the eyes 1000.

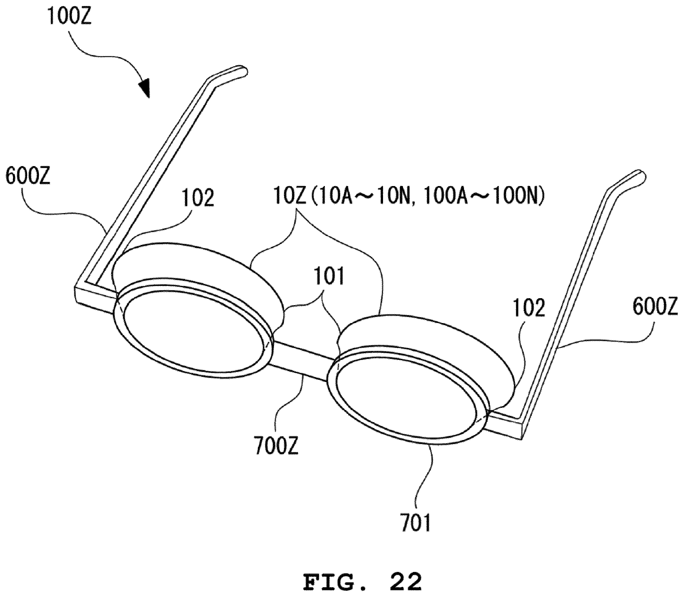

Fig. 22 illustrates a device 100Z according to an embodiment. The device 100Z illustrated in Fig. 22 includes two container bodies 10Z, a connector 700Z, and retainers 600Z. Each container body 10Z may be one of the above-described container bodies 10A to 10N (devices 100A to 100N).

The connector 700Z connects the first ends 101 of the two container bodies 10Z to each other. The first ends 101 of the container bodies 10Z are inner-corner end portions of the container bodies 10Z that are adjacent to the inner corners 1001 when the rims 15 of the two container bodies 10Z are in close contact with the face in the regions around the eyes 1000. The connector 700Z may have a frame shape and include a frame 701 that surrounds the two container bodies 10Z.

The retainers 600Z retain the two container bodies 10Z on the head of the user so that the rims 15 of the container bodies 10Z are in close contact with the face in the regions around the eyes 1000. The retainers 600Z may each have the shape of a temple of eyeglasses. The retainers 600Z extend from the second ends 102 of the two container bodies 10Z. When the connector 700Z includes the frame 701, the retainers 600Z may be connected to the frame 701.

To put the device 100Z on the user, the retainers 600Z are placed on the user's ears while the rims 15 of the two container bodies 10Z are in close contact with the face in the regions around the eyes 1000.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is intended that the following claims define the scope of the invention.

### Reference Signs List

- 10A to 10N, 10X to 10Z: container body
- 11, 11G, 11H, 11J: bottom portion
- 12: peripheral wall
- 13: opening
- 15: rim
- 20A to 20E: partition wall
- 21: outer peripheral edge
- 60A to 60L: irritant placement section
- 60X to 60Z: retainer
- 61: holder body
- 62: connection member
- 70A to 70M: gas-receiving space
- 80A to 80N: tear-fluid-receiving section
- 90: eye irritant
- 91: fiber structure
- 100A to 100N, 100X to 100Z: device
- 101: first end
- 102: second end
- 103: upper end
- 104: lower end
- 111: first section
- 112: second section
- 115: bottom intermediate section
- 117: base
- 121: upper section
- 122: lower section
- 125: peripheral edge
- 131: sealed space
- 220: ventilation hole
- 230: communicating portion
- 300: removably attachable member
- 320: ventilation hole
- 400: tear fluid container
- 401: container opening
- 500: access opening
- 600X to 600Z: retainer
- 700Y, 700Z: connector
- 1000: eye
- 1001: inner corner of eye
- 1002: outer corner of eye
- 1003: upper eyelid
- 1004: lower eyelid
- Da: first direction
- Da1: one side
- Da2: other side
- Db: second direction
- Dc: third direction

## Claims

1. A device for stimulating tear secretion, the device comprising:
a container body (10A-10N, 10X-10Z) including a rim (15) capable of coming into close contact with a face in a region around an eye,
the container body (10A-10N, 10X-10Z) defining therein a gas-receiving space (70A-70M) capable of being filled with gas generated by an eye irritant, **characterised in** the container body (10A-10N, 10X-10z) having an access opening (13) through which an inside of the container body (10A-10N, 10X-10Z) is accessible from an outside when the rim (15) of the container body (10A-10N, 10X-10Z) is in close contact with the face in the region around the eye.

2. The device according to Claim 1, further comprising:
an irritant placement section (60A-60L) that communicates with the gas-receiving space and in which the eye irritant is placed, preferably the irritant placement section is disposed in at least a portion of the gas-receiving space.

3. The device according to Claim 1, further comprising:
a tear-fluid-receiving section (80A-80N) that is provided in the container body and in which tear fluid produced by secretion in the eye is collected.

4. The device according to any one of Claims 1 to 3,
wherein
- the container body (l0A - 10N, l0X - l0Z) has a cup shape with an opening that opens toward one side in a first direction, and
wherein the rim of the container body extends continuously along an entirety of a peripheral edge of the opening; or
- the container body (l0A - 10N, l0X - l0Z) defines a substantially closed space between the container body and the eye when the rim of the container body is in close contact with the face in the region around the eye; or
- the container body (l0A - 10N, l0X - l0Z) includes a bottom portion (11, 11G, 11H, 11J) disposed at other side in the first direction, and
a peripheral wall (12)extending from a peripheral edge of the bottom portion toward the one side in the first direction, preferably further comprising:
a partition wall (20A-20E) that is disposed in the container body (l0A - 10N, l0X - l0Z) and that partitions the gas-receiving space and the tear-fluid-receiving section from each other.

5. The device according to Claim 4,
wherein the container body (10A-10N, 10X-10Z) has a first end and a second end that are respectively positioned adjacent to an outer corner and an inner corner of the eye when the rim (15) of the container body is in close contact with the face in the region around the eye, and
wherein the partition wall (20A-20E) is disposed between the first end and the second end and partitions an interior of the container body into a first section adjacent to the first end and a second section adjacent to the second end,
wherein preferably the first section constitutes the tear-fluid-receiving section and the second section constitutes the gas-receiving space.

6. The device according to Claim 4,
wherein the container body (10A-10N, 10X-10Z) has an upper end and a lower end that are respectively positioned adjacent to an upper eyelid and a lower eyelid when the rim of the container body is in close contact with the face in the region around the eye, and
wherein the partition wall (20A-20E) is disposed between the upper end and the lower end and partitions an interior of the container body into an upper section adjacent to the upper end and a lower section adjacent to the lower end.

7. The device according to Claim 4,
wherein an entirety of an outer peripheral edge (21) of the partition wall is joined to an inner peripheral surface of the container body so that a sealed space is formed between the partition wall and the inner peripheral surface of the container body,
wherein the sealed space constitutes the irritant placement section, and
wherein the partition wall has a ventilation hole through which regions on both sides of the partition wall communicate with each other,
wherein preferably the partition wall has a tubular shape that extends from the bottom portion toward the one side in the first direction, and
wherein the irritant placement section is disposed inside the partition wall having the tubular shape,or
wherein preferably the partition wall is disposed on the peripheral wall at an intermediate position in the first direction and extends along a plane that crosses the first direction to partially partition an inner space of the container body,
wherein the irritant placement section is disposed closer to the bottom portion than the partition wall in the first direction, and
wherein the tear-fluid-receiving section is disposed further away from the bottom portion than the partition wall in the first direction, wherein preferably the partition wall has a communicating portion through which regions closer to and further away from the bottom portion than the partition wall in the first direction communicate with each other in an inner space of the container body, and
wherein the communicating portion is disposed adjacent to an inner corner of the eye when the rim of the container body is in close contact with the face in the region around the eye, and
wherein preferably the partition wall is inclined toward the bottom portion in the first direction from the communicating portion toward the peripheral wall.

8. The device according to Claim 4,
wherein the irritant placement section (60A-60L) is disposed on the bottom portion of the container body.

9. The device according to Claim 8,
wherein the container body (10A-10N, 10X-10Z) has a first end and a second end that are respectively positioned adjacent to an outer corner and an inner corner of the eye when the rim of the container body is in close contact with the face in the region around the eye, and
wherein the bottom portion is inclined toward the other side in the first direction from a bottom intermediate section toward the first end and the second end, the bottom intermediate section being positioned between the first end and the second end,and
wherein preferably the irritant placement section is disposed on the bottom portion at a location closer to the second end than the bottom intermediate section.

10. The device according to Claim 8,
wherein the bottom portion includes a base that bulges toward the one side in the first direction from a peripheral edge of the bottom portion toward an inner region of the bottom portion, and
wherein the irritant placement section (60A-60L) is disposed on the base; or
wherein the bottom portion is removably attachable to the peripheral wall, and
wherein the irritant placement section is disposed on the bottom portion.

11. The device according to Claim 4,
wherein the irritant placement section (60A-60L) is disposed on an inner peripheral surface of the peripheral wall;or
wherein the irritant placement section includes
a holder body that holds the eye irritant, and
a connection member having one end connected to the holder body and other end connected to the container body; or further comprising:
a removably attachable member that is removably attachable to the opening of the container body,
wherein the irritant placement section is disposed on a surface of the removably attachable member, the surface facing an inner region of the container body when the removably attachable member is attached to the opening.

12. The device according to Claim 1, further comprising:
a tear fluid container that is provided integrally with the container body (10A-10N, 10X-10Z) and in which the tear fluid received by the tear-fluid-receiving section is collected,
wherein preferably the tear fluid container has a tubular shape with a bottom that extends outward from the opening of the container body, and
wherein preferably the tear fluid container is disposed on the container body at a location adjacent to an outer corner of the eye when the rim of the container body is in close contact with the face in the region around the eye, and
wherein the tear fluid container has a container opening that opens toward an inner corner of the eye.

13. The device according to Claim 1,
wherein at least a portion or an entirety of the container body (10A-10N, 10X-10Z) is made of a material having transparency characteristics that allow optical observation of an inside of the container body from an outside; or
further comprising:
a fiber structure that supports the eye irritant and that is disposed in the irritant placement section; or
wherein the eye irritant is liquid and sealed in a bag; or
wherein the eye irritant comprises benzyl bromide; or
wherein the eye irritant comprises syn-propanethial-S-oxide; or
wherein the eye irritant comprises at least a portion of an enzyme and a substrate that react to generate the gas.

14. The device according to Claim 13, wherein the enzyme is disposed in the irritant placement section (60A-60L) in a dry state, and
wherein the substrate is added to the enzyme in a form of a solution, and wherein preferably the enzyme comprises alliinase and lachrymatory factor synthase (LFS), and
wherein the substrate is PRENCSO.

15. The device according to Claim 1,
wherein the device comprises:
two of the container bodies; and
a connector that connects inner-corner end portions of the two container bodies to each other, the inner-corner end portions being adjacent to inner corners of the eyes when the rims of the two container bodies are in close contact with the face in the regions around the eyes; or
further comprising:
a retainer (60X-60Z) capable of retaining the or each container body on a head of a subject such that the rim of the container body is in close contact with the face in the region around the eye; or
further comprising:
a retainer (60X-60Z) provided on outer-corner end portions of the two container bodies and capable of retaining the two container bodies on a head of a subject, the outer-corner end portions being adjacent to outer corners of the eyes when the rims of the two container bodies are in close contact with the face in the regions around the eyes.

## Patentansprüche

1. Vorrichtung zum Stimulieren von Tränensekretion, wobei die Vorrichtung umfasst:
einen Behälterkörper (10A-10N, 10X-10Z) mit einem Rand (15), der fähig ist, in einem Bereich um ein Auge in engen Kontakt mit einem Gesicht zu kommen, wobei der Behälterkörper (10A-10N, 10X-10Z) einen Gasaufnahmeraum (70A-70M) darin definiert, der fähig ist, mit von einem Augenreizstoff erzeugtem Gas gefüllt zu werden, **dadurch gekennzeichnet, dass** der Behälterkörper (10A-10N, 10X-10Z) eine Zugangsöffnung (13) aufweist, durch die ein Innenraum des Behälterkörpers (10A-10N, 10X-10Z) von außen zugänglich ist, wenn der Rand (15) des Behälterkörpers (10A-10N, 10X-10Z) in engem Kontakt mit dem Gesicht in dem Bereich um das Auge vorliegt.

2. Vorrichtung nach Anspruch 1, ferner umfassend:
einen Reizstoffplatzierungsabschnitt (60A-60L), der mit dem Gasaufnahmeraum kommuniziert und in dem der Augenreizstoff platziert ist, wobei der der Reizstoffplatzierungsabschnitt vorzugsweise in wenigstens einem Teil des Gasaufnahmeraums angeordnet ist.

3. Vorrichtung nach Anspruch 1, ferner umfassend:
einen Tränenflüssigkeitsaufnahmeabschnitt (80A-80N), der in dem Behälterkörper bereitgestellt ist und in dem durch Sekretion in dem Auge erzeugte Tränenflüssigkeit gesammelt wird.

4. Vorrichtung nach einem der Ansprüche 1 bis 3, wobei
- der Behälterkörper (10A-10N, 10X-10Z) eine Becherform mit einer Öffnung, die sich zu einer Seite in einer ersten Richtung öffnet, aufweist und
wobei sich der Rand des Behälterkörpers durchgehend entlang des gesamten Umfangsrands der Öffnung erstreckt; oder
- der Behälterkörper (10A-10N, 10X-10Z) einen im Wesentlichen geschlossenen Raum zwischen dem Behälterkörper und dem Auge definiert, wenn der Rand des Behälterkörpers in engem Kontakt mit dem Gesicht in dem Bereich um das Auge vorliegt; oder
- der Behälterkörper (10A-10N, 10X-10Z) einen unteren Teil (11, 11G, 11H, 11J) aufweist, der an der anderen Seite in der ersten Richtung angeordnet ist, und
eine Umfangswand (12), die sich von einem Umfangsrand des unteren Teils zu der einen Seite in der ersten Richtung erstreckt, vorzugsweise ferner umfassend:
eine Trennwand (20A-20E), die in dem Behälterkörper angeordnet ist und den Gasaufnahmeraum und den Tränenflüssigkeitsaufnahmeabschnitt voneinander trennt.

5. Vorrichtung nach Anspruch 4,
wobei der Behälterkörper (10A-10N, 10X-10Z) ein erstes Ende und ein zweites Ende aufweist, die jeweils benachbart zu einem äußeren und einem inneren Augenwinkel angeordnet sind, wenn der Rand (15) des Behälterkörpers in engem Kontakt mit dem Gesicht in dem Bereich um das Auge vorliegt, und
wobei die Trennwand (20A-20E) zwischen dem ersten Ende und dem zweiten Ende angeordnet ist und einen Innenraum des Behälterkörpers in einen ersten Abschnitt benachbart zu dem ersten Ende und einen zweiten Abschnitt benachbart zu dem zweiten Ende unterteilt,
wobei vorzugsweise der erste Abschnitt den Tränenflüssigkeitsaufnahmeabschnitt bildet und der zweite Abschnitt den Gasaufnahmeraum bildet.

6. Vorrichtung nach Anspruch 4,
wobei der Behälterkörper (10A-10N, 10X-10Z) ein oberes Ende und ein unteres Ende aufweist, die jeweils benachbart zu einem oberen Augenlid und einem unteren Augenlid angeordnet sind, wenn der Rand des Behälterkörpers in engem Kontakt mit dem Gesicht in dem Bereich um das Auge vorliegt, und
wobei die Trennwand (20A-20E) zwischen dem oberen Ende und dem unteren Ende angeordnet ist und einen Innenraum des Behälterkörpers in einen oberen Abschnitt benachbart zu dem oberen Ende und einen unteren Abschnitt benachbart zu dem unteren Ende unterteilt.

7. Vorrichtung nach Anspruch 4,
wobei der gesamte äußere Umfangsrand (21) der Trennwand mit einer inneren Umfangsfläche des Behälterkörpers verbunden ist, so dass ein abgedichteter Raum zwischen der Trennwand und der inneren Umfangsfläche des Behälterkörpers gebildet wird,
wobei der abgedichtete Raum den Reizstoffplatzierungsabschnitt bildet und
wobei die Trennwand ein Lüftungsloch aufweist, durch das Bereiche auf beiden Seiten der Trennwand miteinander kommunizieren,
wobei vorzugsweise die Trennwand eine rohrförmige Form aufweist, die sich von dem unteren Teil zu der einen Seite in der ersten Richtung erstreckt, und
wobei der Reizstoffplatzierungsabschnitt innerhalb der Trennwand mit der rohrförmigen Form angeordnet ist, oder wobei vorzugsweise die Trennwand an der Umfangswand an einer Zwischenposition in der ersten Richtung angeordnet ist und sich entlang einer Ebene erstreckt, die die erste Richtung kreuzt, um einen Innenraum des Behälterkörpers teilweise zu unterteilen,
wobei der Reizstoffplatzierungsabschnitt in der ersten Richtung näher an dem unteren Teil als die Trennwand angeordnet ist, und
wobei der Tränenflüssigkeitsaufnahmeabschnitt in der ersten Richtung weiter entfernt von dem unteren Teil als die Trennwand angeordnet ist, wobei vorzugsweise die Trennwand einen kommunizierenden Teil aufweist, durch den Bereiche, die in der ersten Richtung näher bei und weiter entfernt von dem unteren Teil als die Trennwand angeordnet sind, in einem Innenraum des Behälterkörpers miteinander kommunizieren, und
wobei der kommunizierende Teil benachbart zu einem inneren Augenwinkel angeordnet ist, wenn der Rand des Behälterkörpers in engem Kontakt mit dem Gesicht in dem Bereich um das Auge vorliegt, und
wobei vorzugsweise die Trennwand in Richtung zu dem unteren Teil in der ersten Richtung von dem kommunizierenden Abschnitt in Richtung zu der Umfangswand geneigt ist.

8. Vorrichtung nach Anspruch 4,
wobei der Reizstoffplatzierungsabschnitt (60A-60L) auf dem unteren Teil des Behälterkörpers angeordnet ist.

9. Vorrichtung nach Anspruch 8,
wobei der Behälterkörper (10A-10N, 10X-10Z) ein erstes Ende und ein zweites Ende aufweist, die jeweils benachbart zu einem äußeren und einem inneren Augenwinkel angeordnet sind, wenn der Rand des Behälterkörpers in engem Kontakt mit dem Gesicht in dem Bereich um das Auge vorliegt, und
wobei der untere Teil zu der anderen Seite in der ersten Richtung von einem unteren Zwischenabschnitt zu dem ersten Ende und dem zweiten Ende hin geneigt ist, wobei der untere Zwischenabschnitt zwischen dem ersten Ende und dem zweiten Ende angeordnet ist, und
wobei vorzugsweise der Reizstoffplatzierungsabschnitt an dem unteren Teil an einer Stelle angeordnet ist, die näher an dem zweiten Ende als der untere Zwischenabschnitt angeordnet ist.

10. Vorrichtung nach Anspruch 8,
wobei der untere Teil eine Basis enthält, die sich zu der einen Seite in der ersten Richtung von einem Umfangsrand des unteren Teils in Richtung zu einem inneren Bereich des unteren Teils wölbt, und
wobei der Reizstoffplatzierungsabschnitt (60A-60L) auf der Basis angeordnet ist; oder
wobei der untere Teil entfernbar an der Umfangswand befestigbar ist, und
wobei der Reizstoffplatzierungsabschnitt auf dem unteren Teil angeordnet ist.

11. Vorrichtung nach Anspruch 4,
wobei der Reizstoffplatzierungsabschnitt (60A-60L) auf einer inneren Umfangsfläche der Umfangswand angeordnet ist; oder
wobei der Reizstoffplatzierungsabschnitt enthält einen Haltekörper, der den Augenreizstoff hält, und
ein Verbindungselement mit einem Ende, das mit dem Haltekörper verbunden ist, und einem anderen Ende, das mit dem Behälterkörper verbunden ist; oder ferner umfassend:
ein entfernbar befestigbares Element, das entfernbar an der Öffnung des Behälterkörpers befestigbar ist,
wobei der Reizstoffplatzierungsabschnitt auf einer Oberfläche des entfernbar anbringbaren Elements angeordnet ist, wobei die Oberfläche einem inneren Bereich des Behälterkörpers zugewandt ist, wenn das entfernbar anbringbare Element an der Öffnung angebracht ist.

12. Vorrichtung nach Anspruch 1, ferner umfassend:
einen Tränenflüssigkeitsbehälter, der integral mit dem Behälterkörper (10A-10N, 10X-10Z) bereitgestellt ist und in dem die von dem Tränenflüssigkeitsaufnahmeabschnitt aufgenommene Tränenflüssigkeit gesammelt wird,
wobei vorzugsweise der Tränenflüssigkeitsbehälter eine rohrförmige Form mit einem Boden aufweist, der sich von der Öffnung des Behälterkörpers nach außen erstreckt, und
wobei vorzugsweise der Tränenflüssigkeitsbehälter an dem Behälterkörper an einer Stelle benachbart zu einem äußeren Augenwinkel angeordnet ist, wenn der Rand des Behälterkörpers in engem Kontakt mit dem Gesicht in dem Bereich um das Auge vorliegt, und
wobei der Tränenflüssigkeitsbehälter eine Behälteröffnung aufweist, die sich zu einem inneren Augenwinkel hin öffnet.

13. Vorrichtung nach Anspruch 1,
wobei wenigstens ein Teil des oder der gesamte Behälterkörper (10A-10N, 10X-10Z) aus einem Material mit Transparenzeigenschaften besteht, die optische Beobachtung eines Inneren des Behälterkörpers von einer Außenseite erlauben; oder
ferner umfassend:
eine Faserstruktur, die den Augenreizstoff trägt und die in dem Reizstoffplatzierungsabschnitt angeordnet ist; oder
wobei der Augenreizstoff flüssig ist und in einem Beutel abgedichtet ist; oder
wobei der Augenreizstoff Benzylbromid umfasst; oder
wobei der Augenreizstoff syn-Propanthial-S-oxid umfasst; oder
wobei der Augenreizstoff wenigstens einen Teil eines Enzyms und ein Substrats umfasst, die reagieren, um das Gas zu erzeugen.

14. Vorrichtung nach Anspruch 13,
wobei das Enzym in dem Reizstoffplatzierungsabschnitt (60A-60L) in einem trockenen Zustand angeordnet ist, und
wobei das Substrat zu dem Enzym in einer Form einer Lösung zugegeben wird und wobei das Enzym vorzugsweise Alliinase und Lachrymatory-Factor-Synthase (LFS) umfasst, und
wobei das Substrat PRENCSO ist.

15. Vorrichtung nach Anspruch 1,
wobei die Vorrichtung umfasst:
zwei der Behälterkörper; und
ein Verbindungselement, das Innenwinkel-Endteile der beiden Behälterkörper miteinander verbindet, wobei die Innenwinkel-Endteile zu inneren Augenwinkeln benachbart sind, wenn die Ränder der beiden Behälterkörper in engem Kontakt mit dem Gesicht in den Bereichen um die Augen vorliegen; oder
ferner umfassend:
eine Haltevorrichtung (60X-60Z), die fähig ist, den oder jeden Behälterkörper an dem Kopf eines Subjekts zu halten, so dass der Rand des Behälterkörpers in engem Kontakt mit dem Gesicht in dem Bereich um das Augevorliegt; oder
ferner umfassend:
eine Haltevorrichtung (60X-60Z), die an Außenwinkel-Endteilen der beiden Behälterkörper bereitgestellt ist und die beiden Behälterkörper an dem Kopf eines Subjekts halten kann, wobei die Außenwinkel-Endteile zu äußeren Augenwinkeln benachbart sind, wenn die Ränder der beiden Behälterkörper in engem Kontakt mit dem Gesicht in den Bereichen um die Augen vorliegen.

## Revendications

1. Dispositif de stimulation de la sécrétion lacrymale, le dispositif comprenant :
un corps de récipient (10A-10N, 10X-10Z) comprenant un bord (15) capable d'entrer en contact étroit avec un visage dans une région autour d'un œil, le corps de récipient (10A-10N, 10X-10Z) définissant à l'intérieur un espace récepteur de gaz (70A-70M) capable d'être rempli de gaz généré par un irritant oculaire, **caractérisé en ce que** le corps de récipient (10A-10N, 10X-10Z) ayant une ouverture d'accès (13) à travers laquelle un intérieur du corps de récipient (10A-10N, 10X-10Z) est accessible de l'extérieur lorsque le bord (15) du corps de récipient (10A-10N, 10X-10Z) est en contact étroit avec le visage dans la région autour de l'œil.

2. Dispositif selon la revendication 1, comprenant en outre :
une section de placement d'irritant (60A-60L) qui communique avec l'espace récepteur de gaz et dans laquelle l'irritant oculaire est placé, de préférence la section de placement d'irritant étant disposée dans au moins une partie de l'espace récepteur de gaz.

3. Dispositif selon la revendication 1, comprenant en outre :
une section de réception de liquide lacrymal (80A-80N) qui est prévue dans le corps de récipient et dans laquelle le liquide lacrymal produit par sécrétion dans l'œil est recueilli.

4. Dispositif selon l'une quelconque des revendications 1 à 3,
dans lequel
- le corps de récipient (10A-10N, 10X-10Z) ayant une forme de coupe avec une ouverture qui s'ouvre vers un côté dans une première direction, et
le bord du corps de récipient s'étendant de manière continue le long de la totalité d'un bord périphérique de l'ouverture ; ou
- le corps de récipient (10A-10N, 10X-10Z) définissant un espace sensiblement fermé entre le corps de récipient et l'œil lorsque le bord du corps de récipient est en contact étroit avec le visage dans la région autour de l'œil ; ou
- le corps de récipient (10A-10N, 10X-10Z) comprenant une partie inférieure (11, 11G, 11H, 11J) disposée sur l'autre côté dans la première direction, et
une paroi périphérique (12) s'étendant à partir d'un bord périphérique de la partie inférieure vers le côté dans la première direction, comprenant de préférence en outre :
une paroi de séparation (20A-20E) qui est disposée dans le corps de récipient et qui sépare l'espace récepteur de gaz et la section de réception de liquide lacrymal l'un de l'autre.

5. Dispositif selon la revendication 4,
le corps de récipient (10A-10N, 10X-10Z) ayant une première extrémité et une seconde extrémité qui sont respectivement positionnées de manière adjacente à un coin extérieur et à un coin intérieur de l'œil lorsque le bord (15) du corps de récipient est en contact étroit avec le visage dans la région autour de l'œil, et
la paroi de séparation (20A-20E) étant disposée entre la première extrémité et la seconde extrémité et séparant un intérieur du corps de récipient en une première section adjacente à la première extrémité et une seconde section adjacente à la seconde extrémité,
de préférence, la première section constituant la section de réception de liquide lacrymal et la seconde section constituant l'espace récepteur de gaz.

6. Dispositif selon la revendication 4,
le corps de récipient (10A-10N, 10X-10Z) ayant une extrémité supérieure et une extrémité inférieure qui sont respectivement positionnées de manière adjacente à une paupière supérieure et à une paupière inférieure lorsque le bord du corps de récipient est en contact étroit avec le visage dans la région autour de l'œil, et
la paroi de séparation (20A-20E) étant disposée entre l'extrémité supérieure et l'extrémité inférieure et séparant l'intérieur du corps de récipient en une section supérieure adjacente à l'extrémité supérieure et une section inférieure adjacente à l'extrémité inférieure.

7. Dispositif selon la revendication 4,
la totalité d'un bord périphérique extérieur (21) de la paroi de séparation étant reliée à une surface périphérique intérieure du corps de récipient de sorte qu'un espace étanche est formé entre la paroi de séparation et la surface périphérique intérieure du corps de récipient,
l'espace étanche constituant la section de placement d'irritant, et
la paroi de séparation ayant un orifice de ventilation à travers lequel des régions situées de part et d'autre de la paroi de séparation communiquent entre elles,
de préférence, la paroi de séparation ayant une forme tubulaire qui s'étend de la partie inférieure vers le côté dans la première direction, et
la section de placement d'irritant étant disposée à l'intérieur de la paroi de séparation de forme tubulaire, ou
de préférence, la paroi de séparation étant disposée sur la paroi périphérique à une position intermédiaire dans la première direction et s'étendant le long d'un plan qui traverse la première direction pour séparer partiellement un espace intérieur du corps de récipient,
la section de placement d'irritant étant disposée plus près de la partie inférieure que la paroi de séparation dans la première direction, et
la section de réception de liquide lacrymal étant disposée plus loin de la partie inférieure que la paroi de séparation dans la première direction, de préférence la paroi de séparation ayant une partie communicante à travers laquelle les régions plus proches et plus éloignées de la partie inférieure que la paroi de séparation dans la première direction communiquent les unes avec les autres dans un espace intérieur du corps de récipient, et
la partie communicante étant disposée de manière adjacente à un coin intérieur de l'œil lorsque le bord du corps de récipient est en contact étroit avec le visage dans la région autour de l'œil, et
de préférence, la paroi de séparation étant inclinée vers la partie inférieure dans la première direction, de la partie communicante vers la paroi périphérique.

8. Dispositif selon la revendication 4,
la section de placement d'irritant (60A-60L) étant disposée sur la partie inférieure du corps de récipient.

9. Dispositif selon la revendication 8,
le corps de récipient (10A-10N, 10X-10Z) ayant une première extrémité et une seconde extrémité qui sont respectivement positionnées de manière adjacente à un coin extérieur et à un coin intérieur de l'œil lorsque le bord du corps de récipient est en contact étroit avec le visage dans la région autour de l'œil, et
la partie inférieure étant inclinée vers l'autre côté dans la première direction à partir d'une section intermédiaire inférieure vers la première extrémité et la seconde extrémité, la section intermédiaire inférieure étant positionnée entre la première extrémité et la seconde extrémité, et
de préférence, la section de placement d'irritant étant disposée sur la partie inférieure à un emplacement plus proche de la seconde extrémité que la section intermédiaire inférieure.

10. Dispositif selon la revendication 8,
la partie inférieure comprenant une base qui se renfle vers le côté dans la première direction à partir d'un bord périphérique de la partie inférieure vers une région intérieure de la partie inférieure, et
la section de placement d'irritant (60A-60L) étant disposée sur la base ; ou
la partie inférieure pouvant être fixée de manière amovible à la paroi périphérique, et
la section de placement d'irritant étant disposée sur la partie inférieure.

11. Dispositif selon la revendication 4,
la section de placement d'irritant (60A-60L) étant disposée sur
une surface périphérique intérieure de la paroi périphérique ; ou
la section de placement d'irritant comprenant
un corps de support qui contient l'irritant oculaire, et un élément de connexion dont une extrémité est reliée au corps du support et l'autre extrémité au corps de récipient ; ou comprenant en outre :
un élément amovible qui peut être fixé de manière amovible à l'ouverture du corps de récipient,
la section de placement d'irritant étant disposée sur une surface de l'élément amovible, la surface faisant face à une région intérieure du corps de récipient lorsque l'élément amovible est fixé à l'ouverture.

12. Dispositif selon la revendication 1, comprenant en outre :
un récipient pour le liquide lacrymal qui est fourni de manière intégrée avec le corps de récipient (10A-10N, 10X-10Z) et dans lequel le liquide lacrymal reçu par la section de réception de liquide lacrymal est recueilli,
de préférence, le récipient de liquide lacrymal ayant une forme tubulaire avec une partie inférieure qui s'étend vers l'extérieur à partir de l'ouverture du corps de récipient, et
de préférence, le récipient de liquide lacrymal étant disposé sur le corps de récipient à un emplacement adjacent à un coin extérieur de l'œil lorsque le bord du corps de récipient est en contact étroit avec le visage dans la région autour de l'œil, et
le récipient de liquide lacrymal ayant une ouverture de récipient qui s'ouvre vers le coin intérieur de l'œil.

13. Dispositif selon la revendication 1,
au moins une partie ou la totalité du corps de récipient (10A-10N, 10X-10Z) étant constitué d'un matériau présentant des caractéristiques de transparence qui permettent une observation optique de l'intérieur du corps de récipient depuis l'extérieur ; ou
comprenant en outre :
une structure de fibres qui supporte l'irritant oculaire et qui est disposée dans la section de placement d'irritant ; ou
l'irritant oculaire étant liquide et scellé dans un sac ; ou
l'irritant oculaire comprenant du bromure de benzyle ; ou
l'irritant oculaire comprenant du syn-propanethial-S-oxyde ; ou
l'irritant oculaire comprenant au moins une partie d'une enzyme et d'un substrat qui réagissent pour générer le gaz.

14. Dispositif selon la revendication 13,
l'enzyme étant disposée dans la section de placement d'irritant (60A-60L) à l'état sec, et
le substrat étant ajouté à l'enzyme sous la forme d'une solution, et de préférence l'enzyme comprenant l'alliinase et la synthase du facteur lacrymogène (LFS), et
le substrat étant du PRENCSO.

15. Dispositif selon la revendication 1,
le dispositif comprenant :
deux des corps de récipient ; et
un connecteur qui relie les parties d'extrémité du coin intérieur des deux corps de récipient, les parties d'extrémité du coin intérieur étant adjacentes aux coins intérieurs des yeux lorsque les bords des deux corps de récipient sont en contact étroit avec le visage dans les régions autour des yeux ; ou
comprenant en outre :
un dispositif de retenue (60X-60Z) capable de retenir le ou chaque corps de récipient sur la tête d'un sujet de telle sorte que le bord du corps de récipient est en contact étroit avec le visage dans la région autour de l'œil ; ou
comprenant en outre :
un dispositif de retenue (60X-60Z) prévu sur les parties d'extrémité des coins extérieurs des deux corps de récipient et capable de retenir les deux corps de récipient sur la tête d'un sujet, les parties d'extrémité des coins extérieurs étant adjacentes aux coins extérieurs des yeux lorsque les bords des deux corps de récipient sont en contact étroit avec le visage dans les régions autour des yeux.
